# EUROPÄISCHE PATENTSCHRIFT

(11) **EP 1 796 548 B1**
(45) Veröffentlichungstag und Bekanntmachung des Hinweises auf die Patenterteilung: **11.11.2009**
(21) Anmeldenummer: 05789704.3
(22) Anmeldetag: 28.09.2005
(51) Int. Cl.: A61B 10/00

(54) **IN-VITRO DIAGNOSTIKUM ZUR SPEICHELVOLUMSBESTIMMUNG**
IN-VITRO DIAGNOSTIC REAGENT FOR DETERMINING SALIVA VOLUME
DIAGNOSTIC IN-VITRO PERMETTANT DE DETERMINER UN VOLUME DE SALIVE

(30) Priorität: 06.10.2004 AT 16702004
(43) Veröffentlichungstag der Anmeldung: 20.06.2007
(73) Patentinhaber: Greiner Bio-One GmbH, 4550 Kremsmünster (AT)
(72) Erfinder: KONRAD, Franz, A-4690 Oberndorf bei Schwanenstadt (AT)
(74) Vertreter: Ofner, Clemens
(86) Internationale Anmeldenummer: PCT/AT2005/000389
(87) Internationale Veröffentlichungsnummer: WO 2006/037140

(56) Entgegenhaltungen:
- EP-A- 0 289 761
- WO-A-2004/046693
- US-A- 4 768 238
- US-A- 5 022 409
- US-A- 5 050 616
- US-A- 5 312 009
- US-A- 5 695 929
- US-A1- 2003 040 009
- US-A1- 2003 164 051
- US-A1- 2003 220 601
- US-A1- 2004 087 874
- US-B1- 6 291 178
- BERTOCCHI P ET AL: "Amperometric ammonium ion and urea determination with enzyme-based probes." 1996, BIOSENSORS & BIOELECTRONICS. 1996, VOL. 11, NR. 1-2, PAGE(S) 1 - 10 , XP002379025 ISSN: 0956-5663 Seite 7, Absatz 5
- PATENT ABSTRACTS OF JAPAN Bd. 1997, Nr. 07, 31. Juli 1997 (1997-07-31) & JP 09 072900 A (BROTHER IND LTD), 18. März 1997 (1997-03-18)
- HAGHIGHAT N ET AL: "The status of lactoferrin and total iron binding capacity of human parotid saliva in Sjögren's syndrome." CLINICAL AND EXPERIMENTAL RHEUMATOLOGY. 2003 JUL-AUG, Bd. 21, Nr. 4, Juli 2003 (2003-07), Seiten 485-488, XP008063804 ISSN: 0392-856X

## Beschreibung

Die vorliegende Erfindung betrifft ein Verfahren zum Sammeln von Speichel aus der Mundhöhle zur Detektion eines Analyten, Speichelsammellösung zur Gewinnung von Speichel, Behälter für biologische Flüssigkeiten bestehend aus einem Boden- und einem Deckelteil, jeweils mit einer Außen- und Innenseite, wobei im Deckelteil eine Ausnehmung als Vertiefung angeordnet ist, in welcher eine Transfereinrichtung bestehend aus einer Einrichtung zur Penetration eines Sammelgefäßes an der Außenseite des Deckelteils und ein Fortsatz mit zwei Enden an der Innenseite des Deckelteils angeordnet ist, verschließbares Sammelgefäß zur Aufnahme von biologischen Flüssigkeiten, wie z.B. Blut, mit einer einer biologischen Probe zugewandten Innenseite, Speichelimitationslösung zur Nachahmung der Zusammensetzung von physiologischem Speichel, Kalibratorlösung zur Quantifizierung des Speichelvolumens, Testkit zur Gewinnung von Speichel und deren Verwendungen.

Zur Bestimmung und Analyse klinisch chemischer Parameter wird sowohl in der Human- als auch Veterinärmedizin vorwiegend Blut verwendet. Es existieren allerdings auch andere Körperflüssigkeiten, die den Zustand der Probanden wieder spiegeln. Sie unterscheiden sich in ihrer Zugänglichkeit.

Blut hat als Untersuchungsmaterial die Nachteile einer komplexen Matrix, einer begrenzten Verfügbarkeit, der Erfordernis von Fachkräften zur Probengewinnung und des damit verbundenen Zeitaufwandes, sowie der Belastung des Patienten mit Schmerzen und Stress.

Speichel wird ausschließlich von drei großen und zahlreichen kleineren Speicheldrüsen produziert und vorwiegend in die Mundhöhle abgegeben. Bei der Probengewinnung muss zwischen Drüsenspeichel, der nur von Drüsen gebildet wird und im Allgemeinen aus dem entsprechenden Ausführungsgang entnommen wird und Mischspeichel, der aus der Mundhöhle stammt, unterschieden werden. Drüsenspeichel ist in erster Linie für wissenschaftliche Untersuchungen, die sich mit der Physiologie und Pathologie einer bestimmten Drüsenart und Transportphänomenen befassen, erforderlich, während der Mischspeichel vorwiegend bei diagnostischen Routineuntersuchungen eingesetzt werden kann, die Rückschlüsse auf Blutbestandteile zulassen.

Speichel ist ein leicht und stressfrei gewinnendes Probenmaterial eine Reihe für Analytbestimmungen. Speicheluntersuchungen stellen unter anderem wertvolle Hilfen für Diagnosen und Therapiekontrolle zahlreicher Krankheiten dar. Im Blickpunkt stehen Eiweiße, Hormone, Stoffwechselmetabolite, Elektrolyte und diverse Pharmaka.

Der Einsatz von Speichel bietet eine Reihe von Vorteilen für die klinisch chemische Diagnostik. Für den Probanden ergeben sich eine Vielzahl unterschiedlicher Vorteile bei dem Einsatz von Speichel anstelle von Blut.

Die geringere Belastung des Probanden bedeutet, dass eine nahezu stressfreie Probenentnahme möglich ist. Daher ist die Speicheldiagnostik besonders für alle Studien interessant, bei denen die Hypothalamus-Hypophysen-Nebennierenachse eine Rolle spielt. Es ist allgemein bekannt, dass wiederholte Venenpunktionen den Response vieler dynamischer Funktionstests maskieren können.

Ferner ist die Speicheldiagnostik für alle Studien attraktiv, die mit häufigen Probenentnahmen verbunden sind, wie z.B. die Erfassung von Biorhythmen, Ovulations- und therapeutisches Monitoring.

Außerdem besteht für den Probanden eine geringere Gefahr von artifiziellen Anämien, Infektionen und Thrombosen als bei Blutabnahmen.

Der Einsatz von Speichel weist auch den Vorteil der geringeren Kosten bei der Probenentnahme auf, weil auf Seite des Personals insgesamt vergleichsweise niedrige Kosten anfallen, da der Einsatz von geschulten Ärzten oder Pflegepersonals entfällt. Speichel kann sogar vom Probanden selbst gewonnen werden und anschließend an ein geeignetes Fachlabors versandt werden.

Auch bezüglich der Diagnostik ergeben sich einige Vorteile des Speicheleinsatzes, wie z.B. die Reflexion von nicht proteingebundenen Plasmafraktionen, die Reflexion von zellulären Konzentrationsverhältnissen, kein Volumenverdrängungseffekt durch Lipide und bei einigen Medikamenten eine bessere Korrelation der Speichelkonzentration mit den pharmakologischen Effekten.

Obwohl im Wachzustand ständig Speichel produziert wird, während 24 Stunden etwa 1000 ml, und Speichel über die Mundöffnung leicht abgegeben werden kann, können sich in der Praxis, besonders bei älteren Patienten, auch Probleme bei der Gewinnung von Speichel ergeben.

Aus dem Stand der Technik sind mehrere Methoden bzw. Vorrichtungen zur Gewinnung von Speichel bereits bekannt.

Der Speichelfluss wird im einfachsten Fall durch Kaubewegungen angeregt. Unter manchen Bedingungen reicht diese Stimulation jedoch nicht aus, wie z.B. bei Dialysepatienten, älteren Probanden, insbesondere am Morgen nach dem Aufwachen.

Eine weitere Methode stellt die Speichelsammlung mit einer Salivette® dar, wobei die Gewinnung des Speichels durch Kauen auf einer Watterolle erfolgt. Für die Speichelgewinnung wird die vollgesaugte Watterolle in das Salivetten-Einhängegefäß gesteckt und zentrifugiert. Der vorliegende, wasserklare Speichel steht für die Analyse zur Verfügung. Werden Feststoffträger, wie z.B. Watte, bei der Speichelgewinnung eingesetzt ist zu prüfen, ob die nachzuweisenden Substanzen von dem verwendeten Material adsorbiert werden. Dies ist insbesondere beim Nachweis von Medikamenten von großer Bedeutung.

Der Speichelfluss kann auch durch mechanische Stimulation, wie Kaubewegungen, angeregt werden. Es wird dabei ein Gummi oder Paraffinwachs mit einer standardisierten Größe verabreicht, wobei der Proband über eine gewisse Zeit mit einer Geschwindigkeit von beispielsweise 70 Kaubewegungen/min darauf kauen muss, wobei der Rhythmus der Kaubewegungen mittels Metronom überwacht werden kann und nach 1 bis 2 Min. der in der Mundhöhle angereicherte Speichel gesammelt wird.

Zur Gewinnung des Mischspeichels kann auch die Spuckmethode verwendet werden. Bei diesem Verfahren wird der in der Mundhöhle gesammelte Speichel einfach ausgespuckt. Dieser Vorgang kann in bestimmten Zeitintervallen mehrmals wiederholt werden.

Eine alternative Methode zur Speichelgewinnung ist die Absaugmethode aus der Mundhöhle, wobei über einen Aspirator, der in der Mundhöhle gesammelte Speichel, über einen vordefinierten Zeitraum abgesaugt wird.

Weiters kann der in der Mundhöhle angesammelte Speichel einfach durch Heraustropfen in einem Probengefäß gesammelt werden.

US 505 0 616 offenbart eine Speichel sammellösung nach dem Oberbegriff des Anspruchs 1 und ihre Verwendung.

Allen voran genannten Methoden zur Speichelgewinnung gemeinsam ist allerdings der Nachteil, dass bei Patienten bzw. bei Personen, welche einen geringen Speichelfluss aufweisen, nur eine sehr geringe Speichelmenge gesammelt werden kann, die Gewinnung nicht reproduzierbar, das Volumen kaum quantifizierbar und somit für den Einsatz in der Diagnostik somit problematisch ist.

Aufgabe der vorliegenden Erfindung ist es einzelne Komponenten bzw. eine Methode zur reproduzierbaren Entnahme und Quantifizierung von Speichel für weitere Analysezwecke zur Verfügung zu stellen.

Die Aufgabe wird durch die Merkmale der unabhängigen Ansprüche 1, 16 und 17 gelöst.

Offenbart wird im folgenden ein Verfahren zum Sammeln von Speichel aus der Mundhöhle zur Detektion eines Analyten umfassend die Schritte (a) Reinigung der Mundhöhle, (b) Anregung der Speichelsekretion mit einer Speichelsammellösung, (c) Entfernen des Speichel-Speichelsammellösungsgemisch aus der Mundhöhle und Sammeln in einem Behälter, (d) Überführen des Speichel-Speichelsammellösungsgemisch in ein verschließbares Sammelgefäß, einer Speichelsammellösung, welche aus einem Mittel mit gustatorisch stimulierenden Eigenschaften, einem Indikatorstoff und/oder Agens und Wasser besteht, einem Behälter wobei im zusammengebauten Zustand des Behälters das dem Bodenteil näher liegende Ende des Fortsatzes der Transfereinrichtung einen Abstand von maximal 3 mm vom Bodenteil aufweist, einem verschließbaren Sammelgefäß, wobei eine Lösung mit einem Reagens zur Stabilisierung bzw. Konservierung und einem Reagens zur Desolvatation der biologischen Flüssigkeit bzw. deren Bestandteile enthalten ist, eine Speichelimitationslösung in welcher Natrium-, Kalium bzw. Kalziumverbindungen, humanes Serumalbumin, Harnstoff und Wasser enthalten sind, eine Kalibratorlösung aus Speichelsammellösung und/oder Speichelimitationslösung und Natriumazid, Kaliumbenzoat und/oder Thimerosal® einem Testkit bestehend aus einer Speichelsammellösung und einem Behälter und deren Verwendung. Von Vorteil dabei erweist sich, dass durch ein standardisierbares Verfahren und standardisierbare Komponenten die Reproduzierbarkeit und Verlässlichkeit der Analysen des gewonnenem Speichels gesteigert wird. Durch den Wegfall eines Festphasenträgers bei einem der Analyseschritte bzw. bei einer der verwendeten Komponenten erweist sich zudem von Vorteil, dass keine nachzuweisenden Substanzen adsorbiert werden können. Durch die guten Reproduktionseigenschaften kann die Erfindung auch für eine Verlaufskontrolle verwendet werden, wobei mehrere Speichelproben notwendig sind. Auch ist die Variabilität von Proband zu Proband geringer.

Durch die Speichelsammellösung mit gustatorisch stimulierenden Eigenschaften wird die Sekretion von Speichel erhöht, wodurch eine größere Probenmenge für die Analyse zur Verfügung steht und die Schwankungen mehrerer Speichelabnahmen niedrig gehalten werden.

Von Vorteil erweist sich, dass das Speichel-Speiehelsammellösungsgemisch über eine im oder am Behälter integrierte Transfereinrichtung in das verschließbare Sammelgefäß, überführt wird, wobei kein Kontakt zwischen der Probe und der die Analyse durchführende Person notwendig ist und dadurch das Infektionsrisiko erheblich gesenkt wird.

Durch die Entfernung partikulärer Bestandteile aus dem Speichel-Speichelsammellösungsgemisch über eine Filtrationseinrichtung im Behälter werden sowohl Partikel, welche die Analyten beeinflussen können als auch die Analyse selbst stören können, frühzeitig eliminiert.

Durch die Verwendung eines verschließbaren Sammelgefäßes mit einer Lösung mit konservierenden und reduzierenden Eigenschaften wird einerseits die Stabilität des Speichel-Speichelsammellösungsgemisches und andererseits die Elimination, beispielsweise durch Zentrifugation, von diversen Proteinen, insbesondere Glycoproteinen, welche die anschließende Analyse negativ beeinflussen könnten, verbessert.

Vorteilhafter Weise werden Kalibratorlösungen verwendet, wodurch sowohl eine Quantifizierung des Speichelvolumens als auch des daraus bestimmten Analyten ermöglicht wird.

Durch den Vergleich der Konzentration und/oder Extinktion des Indikatorstoffes und/oder Agens des Speichel-Speichelsammellösungsgemisches mit der Konzentration und/oder Extinktion des Indikatorstoffes und/oder Agens, welche im Speichel vor dessen Entnahme vorliegt, kann vorteilhafter Weise eine Verfälschung des Ergebnisses der Analyse durch störende Komponenten, welche der Proband beispielsweise durch Nahrungsaufnahme oder dergleichen kurz vor der Speichelgewinnung zu sich genommen hat, verhindert werden.

Das im evakuierten Sammelgefäß vorliegende Speichel-Speichelsammellösungsgemisch wird zentrifugiert, und gegebenenfalls vorher gemischt, wodurch störende Bestandteile in das Pellet wandern und der klare Überstand für die Analyse verwendet werden kann um reproduzierbare Ergebnisse zu erhalten.

Von Vorteil erweist sich, dass das Speichel-Speichelsammellösungsgemisch für Analysen verwendet wird, wodurch leicht zugängliches Probenmaterial analysiert werden kann.

Die Analysen können in standardisierten Labormessgeräten durchgeführt werden, wodurch keine aufwendigen Apparaturen und auch kein speziell geschultes Personal für die Bedienung der Apparaturen notwendig ist.

Das Mittel mit gustatorisch stimulierenden Eigenschaften ist aus einer Gruppe von an- und/oder organischen Genusssäuren und/oder deren Salzen oder Mischungen daraus umfassend Phosphorsäure, Milchsäure, Zitronensäure, Ascorbinsäure ausgewählt, wodurch der Proband durch eventuell versehentlich verschluckter Speichelsammellösung nicht gefährdet ist.

Das Mittel mit gustatorischen Eigenschaften kann auch ein Wirkstoff, wie z.B. Pilocarpin, sein, wodurch bereits auf dem Markt befindliche Arzneien eingesetzt werden können, deren Verträglichkeit bereits tausendfach getestet und evaluiert worden ist.

Die Konzentration des Mittels mit gustatorisch stimulierenden Eigenschaften ist aus einem Bereich mit einer unteren Grenze von 0,0005 %, insbesondere 0,01 %, vorzugsweise 1 %, und einer oberen Grenze von 10 %, insbesondere 5 %, vorzugsweise 2 %, ausgewählt, wobei durch die ausgewählten Konzentrationen eine optimale Anregung des Speichelflusses erfolgt und dennoch keine Gefährdung für den Probanden besteht.

Vorteilhaft erweist sich auch, dass als Indikatorstoff ein Farbstoff enthalten ist, wodurch ein einfacher visueller Nachweis des Speichel-Speichelsammellösungsgemisches möglich ist.

Der Farbstoff ist wasserlöslich, wodurch die Herstellung der Speichelsammellösung einfach und somit kostengünstig durchgeführt werden kann.

Der Farbstoff verursacht vorzugsweise eine Gelbfärbung und ist aus einer Gruppe umfassend Tartrazin, Kurkumin, Safran, Chinolingelb, Sunsetgelb FCF, Gelborange S, Cochenille, Karminsäure, Karmin, Carotine, bzw. Mischungen daraus ausgewählt, wodurch das gewonnene Speichel-Speichelsammellösungsgemisch mit bereits in Labors bzw. Arztpraxen zur Verfügung stehenden Geräten gemessen werden kann.

Es kann auch ein Farbstoff aus einer Gruppe umfassend Riboflavin, Riboflavin-5`-phosphat, Chlorophylle und Chlorphylline, kupferhaltige Komplexe von Chlorophyllen und Chlorophyllinen, Zuckerkulör, einfaches Zuckerkulör, Sulfitlaugen-Zuckerkulör, Ammoniak-Zuckerkulör, Ammonsulfit-Zuckerkulör, Pflanzenkohle, Paprika-Extrakt, Capsanthin, Capsorubin, Beetenrot, Betanin, Anthocyane, Eisenoxide und -hydroxide, Azorubin, Carmoisin, Ponceau 4R, Cochenillerot A, Allurarot AC, Patentblau V, Indigotin, Indigokarmin, Brillantblau FCF, Grün S, Brillantschwarz BN, Schwarz PN, Braun HT, Lycopin, Beta-apo-8'-Carotinal (C 30), Beta-apo-8`-Carotinsäure (C 30)-Ethylester, Lutein, Stoffe aus Maillardverbindungen und/oder Mischungen daraus ausgewählt sein, wodurch eine Vielzahl von unterschiedlichen Färbungen erzielt werden können und dadurch eine Farbcodierung ermöglicht wird.

Die Konzentration des Farbstoffes ist aus einem Bereich mit einer unteren Grenze von 0,0001 %, insbesondere 0,005 %, vorzugsweise 0,1 %, und einer oberen Grenze von 5 %, insbesondere 1 %, vorzugsweise 0,5 %, ausgewählt, wodurch die Kosten für den Farbstoff bei der Herstellung der Speichelsammellösung sehr niedrig gehalten werden können.

Bei der Verwendung eines Agens im Sinne eines Medizinprodukts und/oder in-vitro Diagnostikums bzw. einer pharmazeutischen Substanz erweist sich von Vorteil, dass Stoffe verwendet werden, deren Unbedenklichkeit bereits in klinischen Studien evaluiert wurde bzw. dazu dienen Informationen über physiologische und pathologische Zustände zu liefern.

Vorteilhafter Weise wird ein Agens, welches mit routinemäßig verwendeten Labormessgeräten bestimmt werden kann, beigemengt, wodurch die Analyse in jedem normalen Labor durchgeführt werden kann.

Als Agens ist zumindest ein Vertreter ausgewählt aus einer Gruppe umfassend Bilirubin, Acetaminophen, saure Phosphatase, Albumin, Kreatinin, Alkohol, alkalische Phosphatase, Alaninaminotransferase, Ammoniak, Amylase, Aspartataminotransferase, Bilirubin, unkonjugiert und konjugiert, Harnstoff, Calcium, Bicarbonat, Cholinesterase, LDL- bzw. HDL Cholesterin, Lysozym, Amylase, Creatinkinase, Creatinkinase - MB, Chlorid, Carbamazepin, Creatinin, C-reaktives Protein, direktes Bilirubin, Digoxin, Kohlendioxid, Eisen, Gamma-Glutamyltransferase, Glukose, magnetisches HDL-Cholesterinreagens, Kalium, Lactat, Lactatdehydrogenase, Lithium, Lipase, anorganische Verbindungen, wie z.B. Spurenelemente, Mineralstoffe, wie Magnesium, Natrium, Neugeborenen-Bilirubin, Phenobarbital, Phosphor, Phenytoin, Primidon, Liquorprotein, Salicylat, Gesamt-Bilirubin, Theophyllin, Reagens für totale Eisenbindungskapazität, Gesamtprotein, Triglycerid, Urinprotein, Harnsäure, β-Microglobulin, Corticosteroid- bzw. Sexualhormon bindendes Globulin, Thiocyanat, Transferrin, diverse Lipide, Lipoproteine, Proteine, Kohlenhydrate, insbesondere wasserlösliche Kohlenhydrate, Vitamine, insbesondere wasserlösliche Vitamine, wie z.B. Vitamine der B-, C- und F-Gruppe, anorganische Verbindungen, wie z.B. Spurenelemente, Mineralstoffe, Hormone, wie z.B. Aldosteron, Androstenedion, Cortisol, Dihydroepiandrosteron, bzw. -sulfate, Östradiol, Östriol, Progesteron, Testosteron, bzw. Mischungen daraus zugesetzt, womit durch das Lebensmittelrecht zugelassene Agentien zur Herstellung der Speichelsammellösung verwendet werden und somit deren Wirkung bereits als unbedenklich eingestuft wurde.

Die Konzentration des Agens ist aus einem Bereich mit einer unteren Grenze 0,0000000001 %, insbesondere 0,00005 %, vorzugsweise 0,001 %, und einer oberen Grenze von 10 %, insbesondere 5 %, vorzugsweise 2 %, ausgewählt, wodurch eine physiologisch unbedenkliche Konzentration verwendet wird.

In einer Weiterbildung kann ein Aromastoff und/oder Geschmacksverstärker zugesetzt sein, wodurch sich der Geschmack der Speichelsammellösung verbessern lässt und somit der Speichelfluss zusätzlich angeregt werden kann und das Volumen des sekretierten Speichels erhöht wird.

Der Aromastoff ist aus einer Gruppe von Zuckerarten und/oder Zuckeraustauschstoffen, wie z.B. Saccharose, Maltose, Fructose, und/oder Süßungsmittel, wie z.B. Saccharin, Aspartam, und/oder Mischungen daraus ausgewählt, wodurch einerseits nicht zuckerkranke Personen auf den für sie gewohnten Geschmack nicht verzichten müssen und andererseits auch Diabetiker diese Speichelsammellösung bedenkenlos gurgeln können ohne bei Verschlucken einen Insulinschock befürchten zu müssen.

Der Aromastoff und/oder Geschmacksverstärker natürlichen und/oder synthetischen Ursprungs ist aus einer Gruppe umfassend Früchte, wie Apfel, Himbeere, Kirsche, Erdbeere, Zitrone, Limone, Orange, Lakritze, Kräuter, Chinin, Koffein, Teein, bzw. Mischungen daraus ausgewählt, wodurch Stoffe verwendet werden, auf welche der Mensch bereits konditioniert ist und damit angenehme Empfindungen bzw. Geschmackserlebnisse assoziiert.

Die Konzentration des Aromastoffes und/oder Geschmacksverstärkers ist aus einem Bereich mit einer unteren Grenze von 0,001 %, insbesondere 0,01 %, vorzugsweise 0,1 %, und einer oberen Grenze von 10 %, insbesondere 5 %, vorzugsweise 1 %, ausgewählt, wodurch eine optimale Geschmacksabstimmung erfolgen kann und somit die Akzeptanz für die Speichelsammellösung steigt.

Der pH-Wert ist aus einem Bereich mit einer unteren Grenze von 3, vorzugsweise 3,5, insbesondere 4 und einer oberen Grenze von 6, vorzugsweise 5, insbesondere 4,5 ausgewählt, wodurch die Speichelsekretion optimal angeregt wird und zudem der pH-Wert sinkt, d.h. sich dem Plasma-pH-Wert nähert und somit geringere Schwankungen zeigt.

Der Abstand des dem Bodenteil zugewandten Endes ist aus einem Bereich mit einer unteren Grenze von 0,1 mm, insbesondere 0,5 mm, vorzugsweise 1 mm, und einer oberen Grenze von 3 mm, insbesondere 2 mm, vorzugsweise 1,5 mm, ausgewählt, wodurch äußerst geringe Volumina noch transferiert werden können und somit auch mit wenig Speichel-Speichelsammellösungsgemisch noch eine hochwertige Analyse durchgeführt werden kann.

In einer Weiterbildung des Behälters ist vorgesehen, dass vor oder in der Transfereinrichtung eine Filtrationseinrichtung angeordnet ist, wodurch die Analyse störende Partikel entfernt werden.

Die Filtrationseinrichtung ist aus einem Material gebildet ausgewählt aus einer Gruppe umfassend Glaswolle, Watte, Filterpapier, Schaumgummi, regenerierter Zellulose, Zellulosetriacetat, Nylon, Nitrocellulose, Polyvinyldifluorid (PVDF), PVP, Polyethersulfonat, die sehr gute Filtrationseigenschaften für biologische Proben aufweisen.

In einer alternativen Ausführungsform kann die Filtrationseinrichtung in Form einer Membran angeordnet sein, wodurch diese direkt in die Transfereinrichtung integriert werden kann und kein zusätzlicher bzw. nur minimaler Raum für deren Anordnung benötigt wird.

Vorteilhafter Weise weist die Filtrationseinrichtung eine Porengröße ausgewählt aus einem Bereich mit einer unteren Grenze von 1 µm, insbesondere 10 µm, vorzugsweise 20 µm, und einer oberen Grenze von 100 µm, insbesondere 60 µm, vorzugsweise 50 µm auf, wodurch einerseits der Großteil der verunreinigenden Partikel des Speichel-Speichelsammellösungsgemisches eliminiert werden und andererseits die Durchlässigkeit ohne Verstopfungsgefahr gewährleistet ist.

Das Reagens zur Stabilisierung bzw. Konservierung kann Kaliumbenzoat, Kaliumsorbat, Natriumazid und/oder Thimerosal® sein, wodurch das Speichel-Speichelsammellösungsgemisch über einen längeren Zeitraum aufbewahrt werden kann ohne durch bakterielle oder enzymatische Wirkung die Haltbarkeit der Analyten zu gefährden.

Die Konzentration von Kaliumbenzoat bzw. Kaliumsorbat ist aus einem Bereich mit einer unteren Grenze von 0,05 %, insbesondere 0,1 %, vorzugsweise 1 %, und einer oberen Grenze von 10 %, insbesondere 5 %, vorzugsweise 2 %, ausgewählt und die Konzentration von Natriumazid bzw. Thimerosal® ist aus einem Bereich mit einer unteren Grenze von 0,005 %, insbesondere 0,01 %, vorzugsweise 0,1 %, und einer oberen Grenze von 1 %, insbesondere 0,5 %, vorzugsweise 0,2 %, ausgewählt, wodurch die beste Wirkung für die gewünschten Effekte, nämlich die Stabilisierung bzw. Konservierung erzielt wird.

Das Reagens zur Desolvatation ist Ammoniumsulfat, Natrium-, Kalium- und/oder Kalziumchlorid, wodurch beispielsweise Veränderungen der Oberflächeneigenschaften von Proteinen, insbesondere Glycoproteinen und Proteoglykanen, erzielt werden und diese Proteine dadurch mittels Zentrifugation eliminiert werden können ohne die anschließende Analyse zu stören.

Die Konzentration des Reagens zur Desolvatation ist aus einem Bereich mit einer unteren Grenze von 0,005 %, insbesondere 0,01 %, vorzugsweise 0,1 %, und einer oberen Grenze von 5 %, insbesondere 1 %, vorzugsweise 0,5 %, ausgewählt, wodurch zwar reduzierende Effekte eintreten aber die Analyten dennoch nicht verändert werden, wobei die Analyse verfälscht werden könnte.

Das Volumen der Lösung ist aus einem Bereich mit einer unteren Grenze von 5 µl, insbesondere 20 µl, vorzugsweise 30 µl, und einer oberen Grenze von 500 µl, insbesondere 100µl, vorzugsweise 50 µl, ausgewählt, wobei eine optimale Beschichtung der Innenseite eines 9,5 ml Sammelgefäßes erzielt werden kann.

In einer Weiterbildung ist die Innenseite des Sammelgefäßes mit der Lösung besprüht und gegebenenfalls getrocknet, wodurch gewährleistet wird, dass das Gesamtvolumen nicht verändert wird und somit eine fehlerlose Quantifizierung der Speichelmenge möglich ist.

Von Vorteil zeigt sich auch, dass das Sammelgefäß evakuiert ist, weil dadurch das Speichel-Speichelsammellösungsgemisch selbsttätig vom Behälter in das Sammelgefäß transferiert wird und somit keine Gefährdung für die, die Probengewinnung oder Analyse durchführende Person besteht.

Die Konzentration der Natrium-, Kalium bzw. Kalziumverbindungen der Speichelimitationslösung ist aus einem Bereich mit einer unteren Grenze von 0,001 %, insbesondere 0,01 %, vorzugsweise 0,1 %, und einer oberen Grenze von 20 %, insbesondere 10 %, vorzugsweise 5 %, die von humanem Serumalbumin aus einem Bereich mit einer unteren Grenze von 0,001 %, insbesondere 0,01 %, vorzugsweise 0,1 %, und einer oberen Grenze von 5 %, insbesondere 2,5 %, vorzugsweise 1 %, und die von Harnstoff aus einem Bereich mit einer unteren Grenze von 0,0005 %, insbesondere 0,001 %, vorzugsweise 0,01 %, und einer oberen Grenze von 2 %, insbesondere 1 %, vorzugsweise 0,5 %, ausgewählt, wodurch die physiologischen Konzentrationen des Speichels nachgeahmt werden und dadurch ein Vergleich mit nativem Speichel möglich ist.

In einer Weiterbildung ist Lactoferrin in einer Konzentration aus einem Bereich mit einer unteren Grenze von 0,000001 %, insbesondere 0,00001 %, vorzugsweise 0,0001 %, und einer oberen Grenze von 0,1 %, insbesondere 0,01 %, vorzugsweise 0,005 %, ausgewählt, wodurch eine weitere Komponente, welche in nativem Speichel nachgewiesen werden kann enthalten ist und dadurch die Reproduzierbarkeit erhöht wird.

Zusätzlich kann Gelatine in einer Konzentration ausgewählt aus einem Bereich mit einer unteren Grenze von 0,005 %, insbesondere 0,01 %, vorzugsweise 0,1 %, und einer oberen Grenze von 5 %, insbesondere 1 %, vorzugsweise 0,5 %, enthalten sein, wodurch ebenfalls die native Zusammensetzung des Speichels nachgeahmt wird.

Der pH-Wert der Speichelimitationslösung ist aus einem Bereich mit einer unteren Grenze von 5, vorzugsweise 5,5, insbesondere 6 und einer oberen Grenze von 7,5, vorzugsweise 7, insbesondere 6,5 ausgewählt, eine Optimierung der Quantifizierung erzielt werden kann.

In der Kalibratorlösung sind die Speichelsammellösung und die Speichelimitationslösung in einem annähernd umgekehrt proportionalem Verhältnis enthalten, wodurch eine Quantifizierung möglich ist.

Das Volumen der Speichelsammellösung und der Speichelimitationslösung kann jeweils in einem Gradienten enthalten sein, wodurch eine Quantifizierung ermöglicht wird.

Die Konzentration von Natriumazid, Kaliumbenzoat, Kaliumsorbat und/oder Thimerosal® ist aus einem Bereich mit einer unteren Grenze von 0,005 %, insbesondere 0,01 %, vorzugsweise 0,1 %, und einer oberen Grenze von 5 %, insbesondere 1 %, vorzugsweise 0,5 %, ausgewählt, wobei die Haltbarkeit der Lösung verlängert werden kann.

Der Testkit enthält vorteilhafter Weise eine Speichelsammellösung und einen Behälter, wodurch der Proband ohne medizinisches Fachpersonal die Speichelprobe gewinnen kann.

Durch das verschließbare Sammelgefäß im Testkit wird ermöglicht, dass die Probe versandt werden kann.

Die Speichelimitationslösung und/oder zumindest eine Kalibratorlösung im Testkit ermöglichen eine Quantifizierung des Speichelvolumens.

In einer Weiterbildung des Testkits ist zumindest eine Mikrotiterplatte, Küvette und/oder Gebrauchsanweisung inkludiert, wodurch einerseits standardisierte Labormessgeräte zur Auswertung verwendet werden können und andererseits auch ungeschultes Personal die Quantifizierung durchführen kann.

Es zeigen:
- Fig. 1: einen Querschnitt durch einen Behälter;
- Fig. 2: ein Ergebnis einer Quantifizerung des Speichelvolumens mit Standardgerade bestimmt mit einem Spektralphotometer.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Die vorliegende Erfindung beschreibt ein Verfahren zum Sammeln und Quantifizieren von Speichel bzw. des Volumen. Zudem werden auch die einzelnen dafür benötigten Komponenten beschrieben.

Im folgenden werden die einzelnen Komponenten des Speichelsammelsystems beschrieben, wobei darauf hingewiesen sei, dass auch jede Komponente für sich bereits eine Erfindung darstellt.

Die Speichelsammellösung besteht aus zumindest einem gustatorischen Stimulans, einem Indikatorstoff und/oder Agens und Wasser. Das gustatorische Stimulans dient zur Anregung der Speichelsekretion. Das gustatorische Stimulans wird von einer an- und/oder organischen Genusssäure und/oder deren Salzen oder von Mischungen daraus gebildet. Als organische Genusssäure kann beispielsweise Ascorbinsäure, Milchsäure, etc. verwendet werden. Vorzugsweise wird Zitronensäure verwendet. Alternativ oder zusätzlich zu den organischen und/oder anorganischen Genusssäuren, wie z.B. Phosphorsäure, kann auch ein Wirkstoff, wie z.B. Pilocarpin, zur Anregung der Speichelsekretion verwendet werden. Von den bereits erwähnten an- und organischen Genusssäuren können auch deren Salze, wie z.B. Natrium- bzw. Kaliumsalze, verwendet werden.

Die Konzentration des Mittels mit gustatorisch stimulierenden Eigenschaften kann aus einem Bereich mit einer unteren Grenze von 0,0005 %, insbesondere 0,01 %, vorzugsweise 1 %, und einer oberen Grenze von 10 %, insbesondere 5 %, vorzugsweise 2 %, ausgewählt sein.

Zudem enthält die Speichelsammellösung einen Indikatorstoff und/oder Agens, wobei der Indikatorstoff, von einem Farbstoff Tartrazin und/oder Chinolingeld, gebildet wird. Nicht erfindungsgemäß kommen sämtliche Farbstoffe im Sinne der Richtlinie 94/36/EG in der jeweils gültigen Fassung in Frage. In einer bevorzugten Ausführungsform wird ein Farbstoff verwendet, welcher wasserlöslich ist und eine Gelbfärbung bewirkt. Als Farbstoffe, die eine Gelbfärbung verursachen, stehen Tartrazin, Kurkumin, Safran, Chinolingelb, Sunsetgelb FCF, Gelborange S, Cochenille, Karminsäure, Karmin, Carotine, bzw. Mischungen davon zur Verfügung.

Es können selbstverständlich auch Farbstoffe verwendet werden, welche eine andere Färbung der Speichelsammellösung bewirken, wie z.B. Riboflavin, Riboflavin-5'-phosphat, Chlorophylle und Chlorphylline, kupferhaltige Komplexe von Chlorophyllen und Chlorophyllinen, Zuckerkulör, einfaches Zuckerkulör, Sulfitlaugen-Zuckerkulör, Ammoniak-Zuckerkulör, Ammonsulfit-Zuckerkulör, Pflanzenkohle, Paprika-Extrakt, Capsanthin, Capsorubin, Beetenrot, Betanin, Anthocyane, Eisenoxide und -hydroxide, Azorubin, Carmoisin, Ponceau 4R, Cochenillerot A, Allurarot AC, Patentblau V, Indigotin, Indigokarmin, Brillantblau FCF, Grün S, Brillantschwarz BN, Schwarz PN, Braun HT, Lycopin, Beta-apo-8`-Carotinal (C 30), Beta-apo-8`-Carotinsäure (C 30)-Ethylester, Lutein, Stoffe aus Maillardverbindungen und/oder Mischungen daraus eingesetzt werden.

Die Konzentration des Farbstoffes ist aus einem Bereich mit einer unteren Grenze von 0,0001 %, insbesondere 0,005 %, vorzugsweise 0,1 %, und einer oberen Grenze von 5 %, insbesondere 1 %, vorzugsweise 0,5 %, ausgewählt, wodurch sichergestellt ist, dass eine Färbung der Speichelsammellösung bewirkt wird, die beispielsweise photometrisch detektiert werden kann.

Unabhängig von der Konzentration des verwendeten Farbstoffes in der Speichelsammellösung wird zusätzlich die Extinktion der Speichelsammellösung bei einer vordefinierten Wellenlänge bestimmt.

Alternativ zum Indikatorstoff bzw. zusätzlich kann ein Agens der Speichelsammellösung beigemengt werden.

Für die Verwendung als Agens kommen Stoffe im Sinne eines Medizinproduktes (gemäß Richtlinie 93/42/EWG in der jeweils gültigen Fassung), in-vitro Diagnostikums (gemäß Richtlinie 98/79/EG in der jeweils gültigen Fassung) und/oder pharmazeutischen Stoffes, insbesondere jener, deren Unbedenklichkeit bereits in klinischen Studien evaluiert wurde, in Frage. Als Agens können auch jene Stoffe verwendet werden, die gemäß Richtlinie 95/2/EG nicht als Farbstoff bzw. Süßungsmittel definiert sind, aber als Lebensmittel verwendet werden dürfen.

Als Agens stehen Bilirubin, Acetaminophen, saure Phosphatase, Albumin, Kreatinin, Alkohol, alkalische Phosphatase, Alaninaminotransferase, Ammoniak, Amylase, Aspartataminotransferase, Bilirubin, unkonjugiert und konjugiert, Harnstoff, Calcium, Bicarbonat, Cholinesterase, LDL- bzw. HDL Cholesterin, Lysozym, Amylase, Creatinkinase, Creatinkinase - MB, Chlorid, Carbamazepin, Creatinin, C-reaktives Protein, direktes Bilirubin, Digoxin, Kohlendioxid, Eisen, Gamma-Glutamyltransferase, Glukose, magnetisches HDL-Cholesterinreagens, Kalium, Lactat, Lactatdehydrogenase, Lithium, Lipase, anorganische Verbindungen, wie z.B. Spurenelemente, Mineralstoffe, wie Magnesium, Natrium, Neugeborenen-Bilirubin, Phenobarbital, Phosphor, Phenytoin, Primidon, Liquorprotein, Salicylat, Gesamt-Bilirubin, Theophyllin, Reagens für totale Eisenbindungskapazität, Gesamtprotein, Triglycerid, Urinprotein, Harnsäure, β-Microglobulin, Corticosteroid- bzw. Sexualhormon bindendes Globulin, Thiocyanat, Transferrin, diverse Lipide, Lipoproteine, Proteine, Kohlenhydrate, insbesondere wasserlösliche Kohlenhydrate, Vitamine, insbesondere wasserlösliche Vitamine, wie z.B. Vitamine der B-, C- und F-Gruppe, anorganische Verbindungen, wie z.B. Spurenelemente, Mineralstoffe, Hormone, wie z.B. Aldosteron, Androstenedion, Cortisol, Dihydroepiandrosteron, bzw. -sulfate, Östradiol, Östriol, Progesteron, Testosteron, bzw. Mischungen daraus zur Verfügung.

Die Konzentration des Agens ist aus einem Bereich mit einer unteren Grenze von 0,0000000001 %, insbesondere 0,00005 %, vorzugsweise 0,001 %, und einer oberen Grenze von 10 %, insbesondere 5 %, vorzugsweise 2 %, ausgewählt.

In einer Weiterbildung der Speichelsammellösung kann auch ein Aromastoff und/oder Geschmacksverstärker hinzugefügt werden, welcher beispielsweise den Zitronensäuregeschmack verstärkt oder die Speichelsammellösung süßt, wie dies z.B. mit Zuckerarten bzw. Zuckeraustauschstoffen, wie z.B. Saccharose, Maltose, Fructose, und/oder Süßungsmittel, wie z.B. Saccharin, Aspartam, und/oder Mischungen daraus erreicht werden kann. Als Süßungsmittel können sämtliche Stoffe im Sinne der Richtlinie 94/3 5/EG in der jeweils gültigen Fassung verwendet werden.

Zur Verbesserung des Geschmacks der Speichelsammellösung können auch Aromastoffe und/oder Geschmacksverstärker sowohl natürlichen als auch synthetischen Ursprungs, welche den Geschmack von Apfel, Himbeere, Kirsche, Erdbeere, Zitrone, Limone, Orange, Lakritze, Kräuter, Chinin, Koffein, Teein, bzw. Mischungen daraus imitieren, hinzugefügt werden.

Die Konzentration des Aromastoffes und/oder Geschmacksverstärkers ist aus einem Bereich mit einer unteren Grenze von 0,001 %, insbesondere 0,01 %, vorzugsweise 0,1 %, und einer oberen Grenze von 10 %, insbesondere 5 %, vorzugsweise 1 %, ausgewählt.

Der pH-Wert der Speichelsammellösung ist aus einem Bereich mit einer unteren Grenze von 3, vorzugsweise 3,5, insbesondere 4 und einer oberen Grenze von 6, vorzugsweise 5, insbesondere 4,5 ausgewählt.

Zur Vermeidung von Kontaminationen wird die Speichelsammellösung mit aus dem Stand der Technik bekannten Methoden sterilisiert, wie z.B. mittels Sterilfiltrieren oder Autoklavieren und gegebenenfalls anschließend aliquotiert. Die Speichelsammellösung wird in bruchsichere Gefäße abgefüllt und luftdicht verschlossen. Vorteilhafterweise wird die Speichelsammellösung zudem lichtgeschützt aufbewahrt.

Die Speichelsammellösung wird in einem Volumen ausgewählt aus einem Bereich mit einer unteren Grenze von 0,5 ml, vorzugsweise 1 ml, insbesondere 2 ml und einer oberen Grenze von 10 ml, vorzugsweise 7 ml, insbesondere 5 ml, zum Spülen der Mundhöhle eingesetzt. Besonders vorteilhaft hat sich ein Volumen von 4 ml der Speichelsammellösung zur Spülung der Mundhöhle erwiesen. Die Spülung der Mundhöhle mit Speichelsammellösung kann selbstverständlich mehrmals wiederholt werden. Vor der Spülung der Mundhöhle mit der Speichelsammellösung wird die Schleimhaut der Mundhöhle beispielsweise durch Gurgeln mit Wasser gereinigt. Vorteilhaft erweist sich auch, wenn vor Spülung der Mundhöhle mit der Speichelsammellösung für einen Zeitraum von ungefähr 30 bis 60 Min. keine Nahrung und auch keine Flüssigkeit aufgenommen wird, um eventuelle Verfälschungen der Analyse zu vermeiden. Um auch solche Fehler ausschließen zu können besteht die Möglichkeit die Konzentration des Indikatorstoffes bzw. Agens, welche vor Abnahme des Speichels in der Mundhöhle des Probanden vorliegt zu bestimmen und dies in der weiteren Analyse beispielsweise durch zu Hilfenahme eines Korrekturfaktors zu berücksichtigen.

Die Speichelsammellösung wird für einen Zeitraum, ausgewählt aus einem Bereich mit einer unteren Grenze von 20 sec, vorzugsweise 30 sec, insbesondere 45 sec, und einer oberen Grenze von 10 min, vorzugsweise 4 min, insbesondere 3 min, in der Mundhöhle belassen. Besonders vorteilhaft hat sich ein Zeitraum von 1 bis 2 min für die Aufbewahrung der Speichelsammellösung in der Mundhöhle erwiesen.

Das so erhaltene Gemisch aus Speichel und Speichelsammellösung oder ein Aliquot davon kann nun direkt für Analysen verwendet werden oder zentrifugiert werden und der Überstand mit aus dem Stand der Technik bekannten Methoden der instrumentellen, nass-chemischen oder immunologischen Analytik bestimmt werden.

Vorzugsweise wird das Speichel-Speichelsammellösungsgemisch in einem Behälter 1 gesammelt und weiter aufbereitet und die Quantifizierung des Volumens bzw. Analyse erst später durchgeführt.

Im folgenden sind mehrere Beispiele der Zusammensetzung einer Speichelsammellösung aufgelistet, wobei an dieser Stelle bemerkt sei, dass die erfindungsgemäße Speichelsammellösung nicht auf die nachfolgenden Beispiele beschränkt ist.

| | |
|---|---|
| Kaliumdihydrogencitrat | 5 g |
| Kaliumcitrat Tribasic Monocitrat | 1 g |
| Chinolitigelb | 50 mg |
| auf ein Volumen von 1000 ml mit destilliertem Wasser auffüllen | |
| einen pH-Wert von 3,5 einstellen | |

| | |
|---|---|
| Natriumdihydrogencitrat | 12 g |
| Natriumcitrat Tribasic Monocitrat | 6 g |
| Tartrazin | 30 mg |
| auf ein Volumen von 1000 ml mit destilliertem Wasser auffüllen | |
| einen pH-Wert von 5,2 einstellen | |

| | |
|---|---|
| Natriumdihydrogencitrat | 7 g |
| Natriumcitrat Tribasic Monocitrat | 2 g |
| Tartrazin | 50 mg |
| auf ein Volumen von 1000 ml mit destilliertem Wasser auffüllen | |
| einen pH-Wert von 4,2 einstellen | |

Nicht erfindungsgemäß:

| | |
|---|---|
| Natriumdihydrogencitrat | 20 g |
| Natriumcitrat Tribasic Monocitrat | 8 g |
| Glucose | 5 g |
| auf ein Volumen von 1000 ml mit destilliertem Wasser auffüllen | |
| einen pH-Wert von 4,0 einstellen | |

| | |
|---|---|
| Ascorbinsäure | 4 g |
| Kurkumin . | 2 g |
| auf ein Volumen von 1000 ml mit destilliertem Wasser auffüllen | |
| einen pH-Wert von 5 einstellen | |

| | |
|---|---|
| Pilocarpin | 6 mg |
| Gelborange S | 200 mg |
| Cholesterin | 100 mg |
| Erdbeergeschmack | 10 mg |
| auf ein Volumen von 1000 ml mit destilliertem Wasser auffüllen | |
| einen pH-Wert von 4,5 einstellen | |

Jeweils alternativ zu Natrium-Dihydrogencitrat bzw. Natrium-Citrattribasicmonocitrat können auch deren Kalium- bzw. Calciumverbindungen verwendet werden.

In einer alternativen Ausführungsform wird die Menge des zugegebenen Farbstoffes durch die Bestimmung der optischen Dichte (Extinktion) der Speichelsammellösung festgelegt. Es wird beispielsweise ein Zielwert einer Extinktion von 2 angestrebt und durch die Zugabe einer entsprechenden Menge des gewünschten Farbstoffes, beispielsweise Tartrazin, wird dieser Zielwert erreicht. Der Zielwert für die optische Dichte der Speichelsammellösung ist aus einem Bereich von einer unteren Grenze von 1,0, vorzugsweise 1,2, insbesondere 1,5 und einer oberen Grenze von 2,8, vorzugsweise 2,6, insbesondere 2,4, ausgewählt.

Die Konzentration des Tartrazin in der Speichelsammellösung wird aus einem Bereich mit einer unteren Grenze von 1 mg/l, vorzugsweise 10 mg/l, insbesondere 50 mg/l und einer oberen Grenze von 500 mg/l, insbesondere 250 mg/l, vorzugsweise 100 mg/l, ausgewählt. Besonders vorteilhaft erweist sich Tartrazin in einer Konzentration von 50 mg/l.

Für die Bestimmung der optischen Dichte (OD), synonym Extinktion, kann eine Wellenlänge, ausgewählt aus einem Bereich mit einer unteren Grenze von 380 nm, vorzugsweise 400 nm, insbesondere 420 nm und einer oberen Grenze von 800 nm, 680 nm, insbesondere 560 nm, verwendet werden. Die optische Dichte der Speichelsammellösung mit Tartrazin wird vorzugsweise bei einer Wellenlänge von 450 nm bestimmt.

Wie bereits in den oben angeführten Beispielen aufgezeigt kann in einer alternativen Ausführungsform der Speichelsammellösung anstelle oder zusätzlich zum Farbstoff ein Agens, wie beispielsweise Glucose, zugesetzt werden. Die Konzentration von Glucose in der Speichelsammellösung wird aus einem Bereich mit einer unteren Grenze von 1 g/l, vorzugsweise 2 g/l, insbesondere 3 g/l und einer oberen Grenze von 12 g/l, vorzugsweise 10 g/l, insbesondere 8 g/l ausgewählt. Besonders vorteilhaft hat sich eine Glucosekonzentration von 4 g/l Speichelsammellösung erwiesen.

Je nach verwendetem Indikatorstoff und/oder Agens in der Zusammensetzung der Speichelsammellösung kann diese somit sowohl unter die Kategorie Medizinprodukt bzw. Pharmazeutikum als auch Lebensmittel fallen.

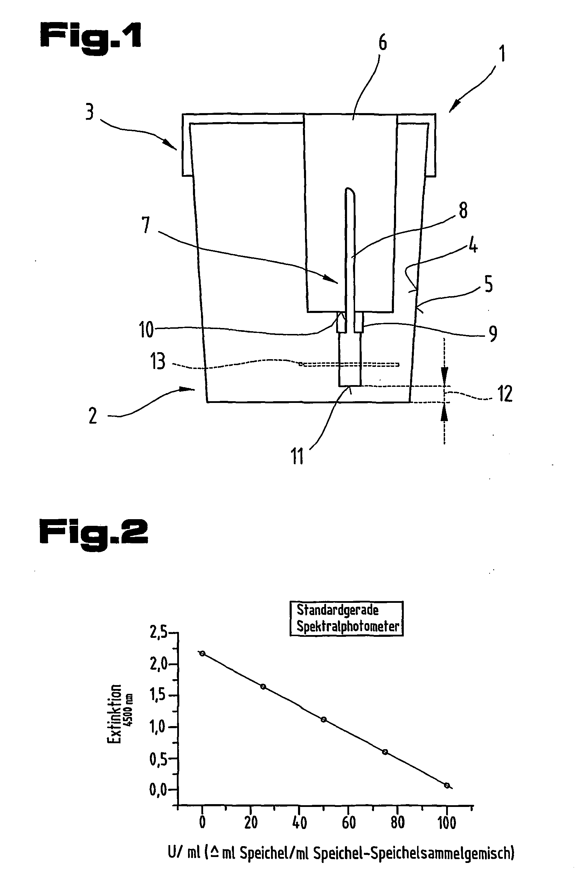

Der Behälter 1 wird im nachfolgenden anhand der in Fig. 1 dargestellten Zeichnung näher erläutert.

Der Behälter 1 in Fig. 1 besteht aus einem Boden- 2 und einem Deckelteil 3, mit jeweils einer Innen- 4 und Außenseite 5, wobei der Bodenteil 2 einen annähernd zylindrischen Becher darstellt.

Der Bodenteil 2 ist vorzugsweise aus Kunststoff, insbesondere Polypropylen, gebildet. Selbstverständlich kann der Bodenteil 2 auch aus anderen Materialien, wie Polystyrol, Polyethylen, gebildet sein.

Der Deckteil 3 besteht ebenfalls aus Kunststoff, vorzugsweise aus Polyethylen, und kann gefärbt sein bzw. aus färbigem Kunststoff hergestellt werden.

Zudem befindet sich im Deckelteil 3 eine Ausnehmung 6, welche eine Vertiefung darstellt, in welcher eine Transfereinrichtung 7 bestehend aus einer Einrichtung zur Penetration 8 eines Sammelgefäßes an der Außenseite 5 des Deckelteils 3 und ein annähernd schlauchförmiger Fortsatz 9 mit zwei Enden, dem oberen Ende 10 und dem unteren Ende 11, an der Innenseite 4 des Deckelteils 3 angeordnet ist. Die Penetrationsvorrichtung 8, wie beispielsweise eine Nadel ist an dem der Außenseite 5 des Deckelteils 3 zugewandten Seite der Transfereinrichtung 7 angeordnet. An jener der Innenseite 4 des Behälters 1 zugewandten Seite ist der annähernd schlauchförmiger Fortsatz 9 in Form einer zylindrischen Röhre ausgebildet. Zwischen dem schlauchförmigen Fortsatz 9, welcher vorzugsweise ebenfalls aus Kunststoff gebildet ist, insbesondere aus Polypropylen, und der Penetrationsvorrichtung 8 befindet sich ein Gewinde oder eine Steckverbindung, um in der Ausnehmung 6 des Deckelteils 3 fixiert werden zu können. Oben erwähnte Behälter 1 aus einem Boden- und einem Deckelteil sind aus dem Stand der Technik, wie z.B. von der Firma Greiner Bio-One, der Vacuette®-Urinbecher, bekannt.

In einer Ausführungsform des Behälters 1 ist der Abstand 12 zwischen dem unteren Ende 11 des schlauchförmigen Fortsatz 9 und dem Boden der Innenseite 4 des Bodenteils 2 aus einem Bereich mit einer unteren Grenze von 0,1 mm, insbesondere 0,5 mm, vorzugsweise 1 mm, und einer oberen Grenze von 3 mm, insbesondere 2 mm, vorzugsweise 1,5 mm, ausgewählt. Besonders vorteilhaft erweist sich ein Abstand 12 von 1 mm. Der geringe Abstand 12 zwischen dem unteren Ende 11 des schlauchförmigen Fortsatz 9 und dem Boden des Bodenteils 2 des Behälters 1 dient vor allem dazu, um auch geringe Volumina bzw.

Restvolumina einer biologischen Flüssigkeit, wie z.B. Urin, Speichel-Speichelsammellösungsgemisch, etc., aus dem Behälter 1 über den schlauchförmigen Fortsatz 9 entfernen zu können. Da in der Analyse des Speichels teilweise auch infektiöse Analyten nachgewiesen werden, ist bei der Verwendung des erfindungsgemäßen Behälters 1 auch von Vorteil, dass die Probe, ohne aus dem Behälter 1 von einer Person entfernt werden zu müssen, direkt in ein neues Gefäß überführt werden kann.

In einer Weiterbildung des Behälters 1 ist vorgesehen, dass eine Filtrationseinrichtung 13 angeordnet ist. Diese Filtrationseinrichtung 13 ist im Bereich der integrierten Transfereinrichtung 7 angeordnet und kann z.B. zwischen der Penetrationsvorrichtung 8 und dem schlauchförmigen Fortsatz 9 der Transfereinrichtung 7 positioniert sein. In einer alternativen Ausführungsform ist die Filtrationseinrichtung 13 dem schlauchförmigen Fortsatz 9 vorgeschaltet bzw. im Verlauf des schlauchförmigen Fortsatz 9 angeordnet. Durch die Filtrationseinrichtung 13 werden partikuläre Bestandteile des Speichel-Speichelsammellösungsgemisches weg filtriert.

Die Filtrationseinrichtung 13 kann aus unterschiedlichen Materialien, wie z.B. Glaswolle, Watte, Filterpapier, Schaumgummi, regenerierter Zellulose, Zellulosetriacetat, Nylon, Nitrocellulose, Polyvinyldifluorid (PVDF), PVP, Polyethersulfonat, bestehen. In einer bevorzugten Ausführungsform wird die Filtrationseinrichtung 13 aus Zellulosetriacetat gebildet.

In einer Weiterbildung der Filtrationseinrichtung 13 ist diese in Form einer Membran im Bereich der Transfereinrichtung 7, insbesondere zwischen der Penetrationsvorrichtung 8 und dem schlauchförmigen Fortsatz 9 angeordnet.

Die Porengröße der Filtrationseinrichtung 13 ist aus einem Bereich mit einer unteren Grenze von 1 µm, insbesondere 10 µm, vorzugsweise 20 µm, und einer oberen Grenze von 100 µm, insbesondere 60 µm, vorzugsweise 50 µm, ausgewählt. Besonders vorteilhaft erweist sich eine Porengröße von 30 µm bis 40 µm.

Über die Penetrationsvorrichtung kann das Speichel-Speichelsammellösungsgemisch in ein erneutes Gefäß übergeführt werden. Dazu eignet sich ein aus dem Stand der Technik bekanntes evakuiertes Sammelgefäßes. Evakuierte Sammelgefäße sind beispielsweise von der Firma Greiner Bio-One unter der Bezeichnung Vacuette® oder von der Firma Becton Dickinson unter der Bezeichnung Vacutainer® auf dem Markt. Durch die Verwendung eines evakuierten Gefäßes wird das Speichel-Speichelsammellösungsgemisch automatisch vom Behälter 1 in das Sammelgefäß überführt.

Das Sammelgefäß enthält eine Lösung bestehend aus Reagenzien, welche der Lösung sowohl konservierende und stabilisierende Eigenschaften, wie z.B. durch Kaliumbenzoat, Natriumazid und/oder Thimerosal® und zudem die Desolvatation ermöglichen, wie z.B. durch Ammoniumsulfat, Natriumchlorid und/oder Kalziumchlorid, verleihen. Hierbei wird eine Reduktion der Hydrathülle und somit eine Änderung der Oberflächeneigenschaften eines Proteins, insbesondere eines Glycoproteins bzw. Proteoglykans bewirkt. Die störenden Proteine können dadurch während eines Zentrifugationsschrittes eliminiert werden, während andere Stoffe, welche in der anschließenden Analyse bestimmt werden sollen, im Überstand bleiben.

Die Konzentration von Kaliumbenzoat ist aus einem Bereich mit einer unteren Grenze von 0,05 %, insbesondere 0,1 %, vorzugsweise 1 %, und einer oberen Grenze von 10 %, insbesondere 5 %, vorzugsweise 2 %, jene von Natriumazid bzw. Thimerosal® aus einem Bereich mit einer unteren Grenze von 0,005 %, insbesondere 0,01 %, vorzugsweise 0,1 %, und einer oberen Grenze von 1 %, insbesondere 0,5 %, vorzugsweise 0,2 %, und jene des Reagens zur Desolvatation ist aus einem Bereich mit einer unteren Grenze von 0,005 %, insbesondere 0,01 %, vorzugsweise 0,1 %, und einer oberen Grenze von 5 %, insbesondere 1 %, vorzugsweise 0,5 %, ausgewählt.

Im folgenden sind mögliche Zusammensetzungen der Lösung, welche sich im Sammelgefäß befindet, angeführt, wobei diese Aufzählung nicht limitierend sein soll.

| | |
|---|---|
| Kaliumbenzoat | 1 g/l |
| Kaliumchlorid | 0,05 g/l |

| | |
|---|---|
| Kaliumbenzoat | 8 g/l |
| Natriumchlorid | 15 g/l |

| | |
|---|---|
| Thimerosal ® | 0,05 g/l |
| Ammoniumsulfat | 0,5 g/l |

| | |
|---|---|
| Natriumazid | 0,5 g/l |
| Ammoniumsulfat | 1,5 g/l |

| | |
|---|---|
| Kaliumsorbat | 0,8 g/l |
| Ammoniumsulfat | 1,2 g/l |

| | |
|---|---|
| Natriumazid | 0,01 g/l |
| Kaliumchlorid | 0,015 g/l |
| Natriumchlorid | 3 g/l |

Vorzugsweise wird die Innenseite eines evakuierten Sammelgefäßes mit der Lösung, beinhaltend eine Auswahl der oben genannten Komponenten, besprüht, um einerseits eine gleichmäßige Beschichtung der Innenseite des Sammelgefäßes zu erzielen und andererseits das Volumen und somit die Konzentration der darin enthaltenen Reagenzien nicht zu verändern.

In einer bevorzugten Ausführungsform wird für das Besprühen der Innenseite des evakuierten Sammelgefäßes ein Gemisch aus einer Lösung beinhaltend Natriumacit in einer Konzentration von 0,5 g/l und Ammoniumsulfat in einer Konzentration von 1, 5 g/l, verwendet.

Für das Besprühen der Innenseite des evakuierten Sammelgefäßes mit einem Fassungsvermögen von annähernd 10 ml wird ein Volumen der Lösung ausgewählt aus einem Bereich mit einer unteren Grenze von 5 µl, vorzugsweise 10 µl, insbesondere 20 µl und einer oberen Grenze von 100 µl, vorzugsweise 80 µl, insbesondere 50 µl, verwendet. Besonders vorteilhaft erweist sich ein Volumen von 30 µl. Selbstverständlich wird falls ein Sammelgefäß mit einem kleineren oder größeren Fassungsvolumen verwendet wird die Menge der Lösung ebenfalls entsprechend vermindert oder erhöht.

In einer Weiterbildung kann die mit der Lösung besprühte Innenseite des Sammelgefäßes auch getrocknet werden.

Alternativ wird die Lösung auch in getrocknetem Zustand in das Sammelgefäß abgefüllt um dadurch das Volumen der darin enthaltenen Flüssigkeiten nicht zu beeinflussen.

Selbstverständlich ist es für einen auf diesem Gebiet tätigen Fachmann auch möglich, unter Verwendung anderer Reagenzien, welche ebenfalls das Abzentrifugieren von zellulären Bestandteilen, insbesondere Glykoproteinen, erleichtern, indem die Oberflächenbeschaffenheit modifiziert wird und mit welchen eine Konservierung bzw. Stabilisierung erzielt werden kann, zu verwenden.

Solche evakuierte Sammelgefäße werden vorzugsweise für den Transfer des Speichel-Speichelsammellösungsgemisch aus dem Behälter 1 verwendet.

Das evakuierte Sammelgefäß mit dem Speichel-Speichelsammellösungsgemisch wird vor der Zentrifugation gekippt, geschüttelt und/oder gevortext, um eine Durchmischung des Speichel-Speichelsammellösungsgemisch mit den sich auf der Innenseite des evakuierten Sammelgefäßes befindlichen Reagenzien zu erzielen. Das Speichel-Speichelsammellösungsgemisch wird im evakuierten Sammelgefäß für ca. 10 min bei ungefähr 1.800 g zentrifugiert. Die Zentrifugation wird so gewählt, dass partikuläre Bestandteile während der Zentrifugation in das Pellet wandern. Der Überstand wird für weitere Analysen herangezogen. Aus dem Überstand kann eine Vielzahl von unterschiedlichen Analyten bestimmt werden.

Der Überstand des evakuierten Sammelgefäß kann nach der Zentrifugation einerseits sofort noch im Sammelgefäß photometrisch gemessen werden oder andererseits kann der Überstand in ein neues Gefäß wie z.B. eine Küvette oder Mikrotiterplatte überführt werden.

Der so gewonnene Überstand kann nun für weiter folgende Analysen beispielsweise in der klinischen Chemie oder für Lateral Flow Immunoassays verwendet werden.

Für eine quantitative Bestimmung des Speichelvolumens wird eine Speichelimitationslösung verwendet. Die Speichelimitationslösung ist eine proteinhältige Lösung, welche synonym auch als synthetischer oder artifizieller Speichel bezeichnet werden kann. Sie besteht zumindest aus Natrium-, Kalium bzw. Kalziumverbindungen, humanem Serumalbumin, Harnstoff und Wasser.

Die Konzentration der Natrium-, Kalium bzw. Kalziumverbindungen ist aus einem Bereich mit einer unteren Grenze von 0,001 %, insbesondere 0,01 %, vorzugsweise 0,1 %, und einer oberen Grenze von 20 %, insbesondere 10 %, vorzugsweise 5 %, jene von humanem Serumalbumin aus einem Bereich mit einer unteren Grenze von 0,001 %, insbesondere 0,01 %, vorzugsweise 0,1 %, und einer oberen Grenze von 5 %, insbesondere 2,5 %, vorzugsweise 1 %, und jene von Harnstoff aus einem Bereich mit einer unteren Grenze von 0,0005 %, insbesondere 0,001 %, vorzugsweise 0,01 %, und einer oberen Grenze von 2 %, insbesondere 1 %, vorzugsweise 0,5 %, ausgewählt.

In einer Weiterbildung der Speichelimitationslösung ist Lactoferrin und/oder Gelatine enthalten um eine möglichst genaue Nachbildung von nativem Speichel zu erzielen. Um die Gelatine zu lösen, wird die Speichelimitationslösung erwärmt, beispielsweise auf 40 °C.

Die Konzentration von Lactoferrin ist aus einem Bereich mit einer unteren Grenze von 0,000001 %, insbesondere 0,00001 %, vorzugsweise 0,0001 %, und einer oberen Grenze von 0,1 %, insbesondere 0,01 %, vorzugsweise 0,005 %, und die Konzentration von Gelatine ist aus einem Bereich mit einer unteren Grenze von 0,005 %, insbesondere 0,01 %, vorzugsweise 0,1 %, und einer oberen Grenze von 5 %, insbesondere 1 %, vorzugsweise 0,5 %, ausgewählt.

Der pH-Wert der Speichelimitationslösung beträgt einen Wert mit einer unteren Grenze von 5, vorzugsweise 5,5, insbesondere 6 und einer oberen Grenze von 7,5, vorzugsweise 7, insbesondere 6,5.

Die Lösung wird mit aus dem Stand der Technik bekannten Methoden sterilisiert, wie z.B. sterifiltriert, und aliquotiert.

Mögliche Zusammensetzungen der Speichelimitationslösung können aus den nachfolgenden Ausführungsbeispielen entnommen werden, wobei darauf hingewiesen sei, diese Aufzählung für die Speichelimitationslösung nicht limitierend sein soll.

| | |
|---|---|
| NaHCO₃ | 0,4 g |
| NaCl | 0,04 g |
| K₂CO₃ | 0,01 g |
| Humanes Serum Albumin | 20 mg |
| Harnstoff | 10 mg |
| mit destilliertem Wasser auf 1000 ml auffüllen | |
| pH-Wert auf 7,5 einstellen | |

| | |
|---|---|
| KHCO₃ | 50 g |
| KCl | 5 g |
| Na₂CO₃ | 3 g |
| Humanes Serum Albumin | 2 g |
| Harnstoff | 1 g |
| mit destilliertem Wasser auf 1000 ml auffüllen | |
| pH-Wert auf 5 einstellen | |

| | |
|---|---|
| NaHCO₃ | 10 g |
| CaCl₂ | 1 g |
| K₂CO₃ | 0,1 g |
| Humanes Serum Albumin | 750 mg |
| Harnstoff | 500 mg |
| Gelatine | 1 g |
| mit destilliertem Wasser auf 1000 ml auffüllen | |
| pH-Wert auf 5,5 einstellen | |

| | |
|---|---|
| NaHCO₃ | 2 g |
| NaCl | 5 g |
| K₂CO₃ | 2 g |
| Humanes Serum Albumin | 2 g |
| Harnstoff | 1 g |
| Lactoferrin | 1 g |
| mit destilliertem Wasser auf 1000 ml auffüllen | |
| pH-Wert auf 7 einstellen | |

| | |
|---|---|
| NaHCO₃ | 0,002 g |
| NaCl | 0,03 g |
| K₂CO₃ | 0,08 g |
| Humanes Serum Albumin | 50 mg |
| Harnstoff | 50 mg |
| Lactoferrin | 0,1 mg |
| Gelatine | 100 mg |
| mit destilliertem Wasser auf 1000 ml auffüllen | |
| pH-Wert auf 6,8 einstellen | |

| | |
|---|---|
| NaHCO₃ | 4,2 g |
| NaCl | 0,5 g |
| K₂CO₃ | 0,2 g |
| Humanes Serum Albumin | 250 mg |
| Harnstoff | 150 mg |
| Lactoferrin | 0,1 g |
| Gelatine | 500 mg |
| mit destilliertem Wasser auf 1000 ml auffüllen | |
| pH-Wert auf 6,5 einstellen | |

In einer Weiterbildung kann die Speichelimitationslösung zur Herstellung von zumindest einer Kalibratorlösung verwendet werden.

Die Kalibratorlösung besteht zumindest aus Speichelsammellösung und/oder eine Speichelimitationslösung und Natriumazid, Kaliumbenzoat, Kaliumsorbat und/oder Thimerosal®.

Vorzugsweise besteht die Kalibratorlösung aus einer Verdünnungsreihe der Speichelimitationslösung bzw. Speichelsammellösung, wobei die beiden Lösungen in einem annähernd umgekehrt proportionalem Verhältnis vorliegen. Die Verdünnungsreihe kann in einem Konzentrationsgradienten von x(n+1) zur Verfügung gestellt werden, wobei x die Anzahl der ml der jeweils verwendeten Lösung und n eine ganze natürliche Zahl ist.

In einer möglichen Ausführungsform umfasst die Verdünnungsreihe fünf unterschiedliche Konzentrationen der Speichelimitationslösung bzw. Speichelsammellösung.

Die Konzentration von Natriumazid, Kaliumbenzoat, Kaliumsorbat und/oder Thimerosal® ist aus einem Bereich mit einer unteren Grenze von 0,005 %, insbesondere 0,01 %, vorzugsweise 0,1 %, und einer oberen Grenze von 5 %, insbesondere 1 %, vorzugsweise 0,5 %, ausgewählt.

Für einen auf diesem Gebiet tätigen Fachmann ist es selbstverständlich möglich mehr oder weniger als fünf Kalibratorlösungen zu verwenden bzw. bei den Verdünnungsreihen das Verhältnis der Speichelimitationslösung und Speichelsammellösung zueinander zu verändern, um eine quantitative Bestimmung des Speichelvolumens durchführen zu können.

Im folgenden sind zur Verdeutlichung der beschriebenen Kalibratorlösungen einige Zusammensetzungen exemplarisch aufgelistet, wobei diese wiederum nicht limitierend sein sollten.

| | Kalibratorlösung 1 | Kalibratorlösung 2 | Kalibratorlösung 3 | Kalibratorlösung 4 | Kalibratorlösung 5 |
|---|---|---|---|---|---|
| Speichelsammellösung | 99,95 % | 74,95 % | 49,975 % | 25 % | 0 % |
| Speichelimitationslösung | 0% | 25 % | 49,975 % | 74,95 % | 99,95 % |
| Natriumazid | 0,05 % | 0,05 % | 0,05 % | 0,05 % | 0,05 % |

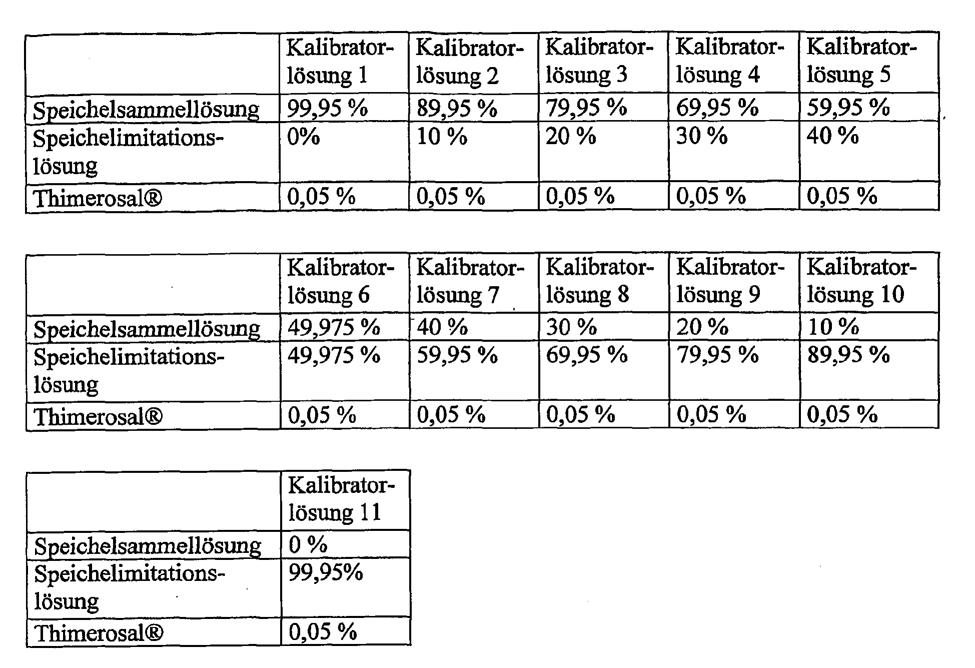

| | Kalibratorlösung 1 | Kalibratorlösung 2 | Kalibratorlösung 3 | Kalibratorlösung 4 |
|---|---|---|---|---|
| Speichelsammellösung | 99,5 % | 66,65 % | 33,3 % | 0 % |
| Speichelimitationslösung | 0% | 33,3 % | 66,65 % | 99,5 % |
| Kaliumbenzoat | 0,5 % | 0,5 % | 0,5 % | 0,5 % |

| | Kalibratorlösung 1 | Kalibratorlösung 2 | Kalibratorlösung 3 | Kalibratorlösung 4 | Kalibratorlösung 5 |
|---|---|---|---|---|---|
| Speichelsammellösung | 99,995 % | 74,995 % | 49,9975 % | 25 % | 0 % |
| Speichelimitationslösung | 0% | 25 % | 49,9975 % | 74,995 % | 99,995 % |
| Natriumazid | 0,005 % | 0,005 % | 0,005 % | 0,005 % | 0,005 % |

Die genau Angabe des Volumens des gewonnenen Speichels ist von größter Bedeutung, weil dadurch berechnet werden kann, welche Menge des in der Folge bestimmten Analyten sich in der ursprünglich entnommenen biologischen Probe befindet.

Durch die Verwendung unterschiedlicher Indikatorstoffe, insbesondere Farbstoffe bzw. Agentien ist es auch möglich, dass die Kalibratorlösungen entsprechend unterschiedlich zusammengesetzt sind. Exemplarisch können neben einer Speichelsammellösung mit Farbstoff auch solche mit Glucose als Kalibratorlösungen eingesetzt werden.

Anstelle der beschriebenen Speichelimitationslösung kann für die Herstellung der Kalibratorlösung bzw. deren Verdünnungsreihen auch destilliertes Wasser verwendet werden. Um allerdings eine bessere Quantifizierbarkeit des Speichelvolumens zu erzielen, wird die Speichelimitationslösung bevorzugt.

Um Kontaminationen zu vermeiden werden die Kalibratorlösungen mit aus dem Stand der Technik bekannten Methoden sterilisiert.

In einer Weiterbildung werden zumindest einige der vorab genannten Komponenten zu einem Testkit zusammengefasst. Der Testkit umfasst zumindest die Speichelsammellösung und einen Behälter 1 zur Aufnahme des Speichel-Speichelsammellösungsgemisch. Der Testkit kann mit einem verschließbaren Sammelgefäß, Speichelimitationslösung, Kalibratorlösung und/oder Kontrolllösungen ergänzt werden. Optional können im Testkit auch Mikrotiterplatten bzw. Küvetten, welche in standardisierten Labormessgeräten ausgewertet werden, und/oder eine Gebrauchsanweisung hinzugefügt werden.

In der im Testkit enthaltenen Null-Kontrolllösung ist Wasser oder Speichelimitationslösung enthalten. Die Null-Kontrolllösung muss eine Extinktion von 0 ergeben. Die Positivkontrolle wird von Wasser oder Speichelimitationslösung mit Farbstoff oder Speichelsammellösung gebildet. Die Positivkontrolle muss eine Extinktion größer 0 ergeben.

In Fig. 2 ist eine Standardgerade eines Spektralphotometers von Kalibratorlösungen 1 bis 5 dargestellt, wobei jeweils 1 ml der Kalibrationslösungen 1 bis 5 in 1 cm Küvetten pipettiert wird und bei einer Wellenlänge von 450 nm in einem Standardspektralphotometer vermessen wird. Auf der X-Achse ist die Prozentangabe des Speichelvolumens, welche den Units Farbstoff/ml Speichel-Speichelsammellösungsgemisch entspricht, pro Milliliter Probe und auf der Y-Achse die Extinktion bei einer Wellenlänge von 450 nm festgehalten.

Durch eine Vergleichsmessung der optischen Dichte bei einer Wellenlänge von 450 nm des Speichel-Speichelsammellösungsgemisches aus dem evakuierten Sammelgefäßes im Vergleich zu den Kalibratorlösungen 1 bis 5, kann nun das Volumen des Speichels pro ml Probe bestimmt werden.

Bei der Weiterverwendung des Speichels aus dem Überstand des evakuierten Sammelgefäßes kann daher bei der Bestimmung, insbesondere der quantitativen Bestimmung, von Analyten die Menge des ursprünglich gewonnenen Speichels berücksichtigt werden.

Selbstverständlich kann die Erfindung nicht nur in der Humanmedizin sondern selbstverständlich mit geringfügigen Änderungen, wie z.B. durch Verwendung des der Tierart entsprechenden Albumins der Speichelimitationslösung, auch in der Veterinärmedizin angewendet werden.

Das Verfahren zum Sammeln von Speichel und der Testkit bzw. die diesen bildenden Komponenten können auch im Homecare-Bereich eingesetzt werden.

### Ausführungsbeispiel 1

Bevor der Proband 2 ml Speichelsammellösung mit der in der in der Tabelle aufgezeigten Zusammensetzung gurgelt, spült er den Mund mit destilliertem Wasser um eventuell in der Mundhöhle vorliegende Speisereste oder andere störende Komponenten zu entfernen.

### Speichelsammellösung:

| | |
|---|---|
| Pilocarpin | 6 mg |
| Gelborange S | 200 mg |
| Cholesterin | 100 mg |
| Erdbeergeschmack | 10 mg |
| auf ein Volumen von 1000 ml mit destilliertem Wasser auffüllen | |
| einen pH-Wert von 4,5 einstellen | |

Nach vier Mal eine Minute Gurgelns mit jeweils 2 ml Speichelsammellösung wird das gesamte Speichel-Speichelsammellösungsgemisch im Behälter 1 gesammelt, wobei der Abstand des schlauchförmigen Fortsatz 9 vom Boden des Bodenteils 2 des Behälters 12 mm beträgt. Zudem ist im Behälter 1 im Bereich des schlauchförmigen Fortsatz 9 eine Filtrationseinrichtung 13 aus Glaswolle angeordnet. Die Porengröße der Filtrationseinrichtung 13 beträgt 50 µm.

Das Speichel-Speichelsammellösungsgemisch wird in ein evakuiertes Sammelgefäß, welches mit der in der Tabelle angeführten Lösung beschichtet ist, transferiert.

### Lösung mit Reagenzien:

| | |
|---|---|
| Natriumazid | 0,01 g/l |
| Kaliumchlorid | 0,015 g/l |
| Natriumchlorid | 3 g/l |

Das Sammelgefäß mit dem darin enthaltenen Speichel-Speichelsammellösungsgemisch wird gevortext und bei 2.000 g für 30 Minuten bei 4 °C zentrifugiert. Das Pellet wird verworfen und die optische Dichte des Überstands wird bei einer Wellenlänge von 480 nm in einem Plattenphotometer bestimmt, wobei 250 µl des Speichel-Speichelsammellösungsgemisch in jeweils ein Well einer Mikrotiterplatte pipettiert wird.

Um eine Quantifizierung zu ermöglichen wird parallel die optische Dichte der Kalibratorlösungen 1 bis 3 mit der in der nachstehenden Tabelle angeführten Zusammensetzung ebenfalls bei einer Wellenlänge von 480 nm gemessen.

### Kalibratorlösungen:

| | Kalibratorlösung 1 | Kalibratorlösung 2 | Kalibratorlösung 3 | Kalibratorlösung 4 |
|---|---|---|---|---|
| Speichelsammellösung | 99,5 % | 66,65 % | 33,3 % | 0 % |
| Speichelimitationslösung | 0% | 33,3 % | 66,65 % | 99,5 % |
| Kaliumbenzoat | 0,5 % | 0,5 % | 0,5 % | 0,5 % |

Die für die Herstellung der Kalibratorlösung verwendete Speichelsammellösung weist die in Ausführungsbeispiel 1 angeführte Zusammensetzung und die Speichelimitationslösung weist die in der nachfolgenden Tabelle angeführte Zusammensetzung auf.

### Speichelimitationslösung:

| | |
|---|---|
| NaHCO₃ | 0,4 g |
| NaCl | 0,04 g |
| K₂CO₃ | 0,01 g |
| Humanes Serum Albumin | 20 mg |
| Harnstoff | 10 mg |
| mit destilliertem Wasser auf 1000 ml auffüllen | |
| pH-Wert auf 7,5 einstellen | |

Für die photometrische Bestimmung wird als Positivkontrolle eine Lösung 1 mit einer Konzentration von 10 U/ml, entspricht einer Konzentration von 10 %, dh 0,1 ml Speichel/ml Probe und einer Lösung 2 mit einer Konzentration von 90 U/ml, entspricht einer Konzentration von 90 %, dh 0,9 ml Speichel/ml Probe verwendet. Das bestimmte Speichelvolumen beträgt 10 ml.

### Ausführungsbeispiel 2

Der Proband gurgelt 6 ml Speichelsammellösung mit der in der in der Tabelle aufgezeigten Zusammensetzung.

### Speichelsammellösung:

| | |
|---|---|
| Ascorbinsäure | 4 g |
| Kurkumin | 2 g |
| auf ein Volumen von 1000 ml mit destilliertem Wasser auffüllen | |
| einen pH-Wert von 5 einstellen | |

Nach 5 Minuten gurgeln mit der Speichelsammellösung wird das Speichel-Speichelsammellösungsgemisch im Behälter 1 gesammelt, wobei der Abstand des schlauchförmigen Fortsatz 9 vom Boden des Bodenteils 2 des Behälters 1 0,9 mm beträgt. Zudem ist im Behälter 1 im Bereich des schlauchförmigen Fortsatz 9 eine Filtrationseinrichtung 13 in Form einer Nitrocellulosemembran zwischen der Penetrationsvorrichtung 8 und dem schlauchförmigen Fortsatz 9 angeordnet. Die Porengröße der Filtrationseinrichtung 13 beträgt 10 µm.

Das Speichel-Speichelsammellösungsgemisch wird in ein evakuiertes Sammelgefäß, welches mit der in der Tabelle angeführten Lösung beschichtet ist, transferiert.

### Lösung mit Reagenzien:

| | |
|---|---|
| Kaliumbenzoat | 8 g/l |
| Natriumchlorid | 15 g/l |

Das Sammelgefäß mit dem darin enthaltenen Speichel-Speichelsammellösungsgemisch wird geschüttelt und bei 1.500 g für 1 Stunde bei Raumtemperatur zentrifugiert. Das Pellet wird verworfen und die optische Dichte des Überstands wird bei einer Wellenlänge von 400 nm in einem Photometer bestimmt.

Um eine Quantifizierung zu ermöglichen wird parallel die optische Dichte der Kalibratorlösungen 1 bis 11 mit der in der nachstehenden Tabelle angeführten Zusammensetzung ebenfalls bei einer Wellenlänge von 400 nm gemessen.

### Kalibratorlösungen:

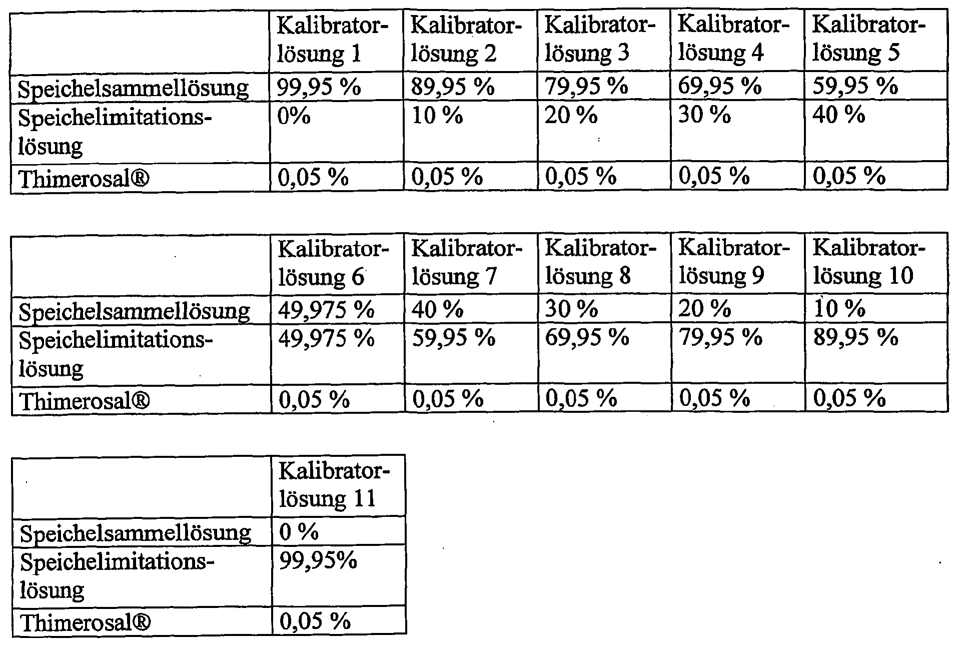

Die für die Herstellung der Kalibratorlösung verwendete Speichelsammellösung weist die in Ausführungsbeispiel 2 angeführte Zusammensetzung und die Speichelimitationslösung weist die in der nachfolgenden Tabelle angeführte Zusammensetzung auf.

### Speichelimitationslösung:

| | |
|---|---|
| NaHCO₃ | 0,002 g |
| NaCl | 0,03 g |
| K₂CO₃ | 0,08 g |
| Humanes Serum Albumin | 50 mg |
| Harnstoff | 50 mg |
| Lactoferrin | 0,01 mg |
| Gelatine | 100 mg |
| mit destilliertem Wasser auf 1000 ml auffüllen | |
| pH-Wert auf 6,8 einstellen | |

Für die photometrische Bestimmung als Positivkontrolle eine Lösung mit 50 U/ml verwendet, das entspricht einer Speichelkonzentration pro ml Probe von 50 %, dh 0,5 ml Speichel/ml Probe. Das entnommene Speichelvolumens dieses Ausführungsexperiments beträgt 2 ml.

### Ausführungsbeispiel 3

Bevor der Proband 5 ml Speichelsammellösung mit der in der in der Tabelle aufgezeigten Zusammensetzung gurgelt, spült er den Mund mit destilliertem Wasser um eventuell in der Mundhöhle vorliegende Speisereste oder andere störende Komponenten zu entfernen.

### Speichelsammellösung:

| | |
|---|---|
| Natriumdihydrogencitrat | 20 g |
| Natriumcitrat Tribasic Monocitrat | 8 g |
| Glucose | 5 g |
| auf ein Volumen von 1000 ml mit destilliertem Wasser auffüllen | |
| einen pH-Wert von 4,0 einstellen | |

Nach drei Minuten Gurgeln mit der Speichelsammellösung wird das Speichel-Speichelsammellösungsgemisch im Behälter 1 gesammelt, wobei der Abstand des schlauchförmigen Fortsatz 9 vom Boden des Bodenteils 2 des Behälters 1 3 Millimeter beträgt.

Das Speichel-Speichelsammellösungsgemisch wird in ein evakuiertes Sammelgefäß, welches mit der in der Tabelle angeführten Lösung beschichtet ist, transferiert.

### Lösung mit Reagenzien:

| | |
|---|---|
| Kaliumbenzoat | 1 g/l |
| Kaliumchlorid | 0,05 g/l |

Das Sammelgefäß mit dem darin enthaltenen Speichel-Speichelsammellösungsgemisch wird gekippt und bei 2.500 g für 3 Minuten bei Raumtemperatur zentrifugiert. Das Pellet wird verworfen und die optische Dichte des Überstands wird bei einer Wellenlänge von 380 nm in einem Spektralphotometer bestimmt.

Um eine Quantifizierung zu ermöglichen wird parallel die optische Dichte der Kalibratorlösungen 1 bis 5 mit der in der nachstehenden Tabelle angeführten Zusammensetzung ebenfalls bei einer Wellenlänge von 450 nm gemessen.

### Kalibratorlösungen:

| | Kalibratorlösung 1 | Kalibratorlösung 2 | Kalibratorlösung 3 | Kalibratorlösung 4 | Kalibratorlösung 5 |
|---|---|---|---|---|---|
| Speichelsammellösung | 99,95 % | 69,95 % | 39,95 % | 20 % | 0 % |
| Speichelimitationslösung | 0% | 30 % | 60 % | 79,95 % | 99,95 % |
| Natriumazid | 0,05 % | 0,05 % | 0,05 % | 0,05 % | 0,05 % |

Die für die Herstellung der Kalibratorlösung verwendete Speichelsammellösung weist die in Ausführungsbeispiel 3 angeführte Zusammensetzung und die Speichelimitationslösung weist die in der nachfolgenden Tabelle angeführte Zusammensetzung auf.

### Speichelimitationslösung:

| | |
|---|---|
| NaHCO₃ | 10 g |
| CaCl₂ | 1 g |
| K₂CO₃ | 0,1 g |
| Humanes Serum Albumin | 750 mg |
| Harnstoff | 500 mg |
| Gelatine | 1 g |
| mit destilliertem Wasser auf 1000 ml auffüllen | |
| pH-Wert auf 5,5 einstellen | |

Für die enzymatisch photometrische Bestimmung als Positivkontrolle Kalibratorlösung 2 und 4 verwendet. Das gemessene Speichelvolumen beträgt 6 ml.

### Ausführungsbeispiel 4

Bevor der Proband 3 ml Speichelsammellösung mit der in der in der Tabelle aufgezeigten Zusammensetzung gurgelt, spült er den Mund mit destilliertem Wasser um eventuell in der Mundhöhle vorliegende Speisereste oder andere störende Komponenten zu entfernen.

### Speichelsammellösung:

| | |
|---|---|
| Natriumdihydrogencitrat | 7 g |
| Natriumcitrat Tribasic Monocitrat | 2 g |
| Tartrazin | 50 mg |
| auf ein Volumen von 1000 ml mit destilliertem Wasser auffüllen | |
| einen pH-Wert von 4,2 einstellen | |

Nach einer Minute gurgeln mit der Speichelsammellösung wird das Speichel-Speichelsammellösungsgemisch im Behälter 1 gesammelt, wobei der Abstand des schlauchförmigen Fortsatz 9 vom Boden des Bodenteils 2 des Behälters 1 einen Millimeter beträgt. Zudem ist im Behälter 1 im Bereich des schlauchförmigen Fortsatz 9 eine Filtrationseinrichtung 13 aus Zellulosetriacetat angeordnet. Die Porengröße der Filtrationseinrichtung 13 beträgt 30 µm.

Das Speichel-Speichelsammellösungsgemisch wird in ein evakuiertes Sammelgefäß, welches mit der in der Tabelle angeführten Lösung beschichtet ist, transferiert.

### Lösung mit Reagenzien:

| | |
|---|---|
| Natriumazid | 0,5 g/l |
| Ammoniumsulfat | 1,5 g/l |

Das Sammelgefäß mit dem darin enthaltenen Speichel-Speichelsammellösungsgemisch wird gekippt und bei 1.800 g für 10 Minuten bei Raumtemperatur zentrifugiert. Das Pellet wird verworfen und die Extinktion des Überstands wird bei einer Wellenlänge von 450 nm in einem Spektralphotometer bestimmt.

Um eine Quantifizierung zu ermöglichen wird parallel die Extinktion der Kalibratorlösungen 1 bis 5 mit der in der nachstehenden Tabelle angeführten Zusammensetzung ebenfalls bei einer Wellenlänge von 450 nm gemessen.

### Kalibratorlösungen:

| | Kalibratorlösung 1 | Kalibratorlösung 2 | Kalibratorlösung 3 | Kalibratorlösung 4 | Kalibratorlösung 5 |
|---|---|---|---|---|---|
| Speichelsammellösung | 99,95 % | 74,95 % | 49,975 % | 25 % | 0 % |
| Speichelimitationslösung | 0% | 25 % | 49,975 % | 74,95 % | 99,95 % |
| Natriumazid | 0,05 % | 0,05 % | 0,05 % | 0,05 % | 0,05 % |

Die für die Herstellung der Kalibratorlösung verwendete Speichelsammellösung weist die in Ausführungsbeispiel 4 angeführte Zusammensetzung und die Speichelimitationslösung weist die in der nachfolgenden Tabelle angeführte Zusammensetzung auf.

### Speichelimitationslösung:

| | |
|---|---|
| NaHCO₃ | 4,2 g |
| NaCl | 0,5 g |
| K₂CO₃ | 0,2 g |
| Humanes Serum Albumin | 250 mg |
| Harnstoff | 150 mg |
| Lactoferrin | 0,1 mg |
| Gelatine | 500 mg |
| mit destilliertem Wasser auf 1000 ml auffüllen | |
| pH-Wert auf 6,5 einstellen | |

Für die photometrische Bestimmung werden zwei Kontrollen verwendet. Kontrolle 1 weist eine Konzentration von 20 U/ml (%) und Kontrolle 2 von 60 U/ml (%) auf, wobei 1 U/ml (%) entspricht 0,01 ml Speichel in der Speichel-Speichelsammellösung. Das entnommene Speichelvolumen beträgt 3 ml.

### Ausführungsbeispiel 5

Bevor der Proband jeweils 1,5 ml Speichelsammellösung mindestens 4 mal mit der in der in der Tabelle aufgezeigten Zusammensetzung gurgelt, spült er den Mund mit destilliertem Wasser um eventuell in der Mundhöhle vorliegende Speisereste oder andere störende Komponenten zu entfernen.

### Speichelsammellösung:

| | |
|---|---|
| Natriumdihydrogencitrat | 12 g |
| Natriumcitrat Tribasic Monocitrat | 6 g |
| Tartrazin | 30 mg |
| auf ein Volumen von 1000 ml mit destilliertem Wasser auffüllen | |
| einen pH-Wert von 5,2 einstellen | |

Nach jeweils einer Minute Gurgeln mit der Speichelsammellösung wird das Speichel-Speichelsammellösungsgemisch im Behälter 1 gesammelt, wobei der Abstand des schlauchförmigen Fortsatz 9 vom Boden des Bodenteils 2 des Behälters 12 mm beträgt. Zudem ist im Behälter 1 im Bereich des schlauchförmigen Fortsatz 9 eine Filtrationseinrichtung 13 aus Watte angeordnet. Die Porengröße der Filtrationseinrichtung 13 beträgt 70 µm.

Das Speichel-Speichelsammellösungsgemisch wird in ein evakuiertes Sammelgefäß, welches mit der in der Tabelle angeführten Lösung beschichtet ist, transferiert.

### Lösung mit Reagenzien:

| | |
|---|---|
| Thimerosal ® | 0,05 g/l |
| Ammoniumsulfat | 0,5 g/l |

Das Sammelgefäß mit dem darin enthaltenen Speichel-Speichelsammellösungsgemisch wird gekippt und bei 1.500 g für 20 Minuten bei 4 °C zentrifugiert. Das Pellet wird verworfen und die optische Dichte des Überstands wird bei einer Wellenlänge von 420 nm in einem Spektralphotometer bestimmt.

Um eine Quantifizierung zu ermöglichen wird parallel die optische Dichte der Kalibratorlösungen 1 bis 5 mit der in der nachstehenden Tabelle angeführten Zusammensetzung ebenfalls bei einer Wellenlänge von 420 nm gemessen.

### Kalibratorlösungen:

| | Kalibratorlösung 1 | Kalibratorlösung 2 | Kalibratorlösung 3 |
|---|---|---|---|
| Speichelsammellösung | 99,95 % | 49,975 % | 0 % |
| Speichelimitationslösung | 0% | 49,975 % | 99,95 % |
| Natriumazid | 0,05 % | 0,05 % | 0,05 % |

Die für die Herstellung der Kalibratorlösung verwendete Speichelsammellösung weist die in Ausführungsbeispiel 5 angeführte Zusammensetzung und die Speichelimitationslösung weist die in der nachfolgenden Tabelle angeführte Zusammensetzung auf.

### Speichelimitationslösung:

| | |
|---|---|
| NaHCO₃ | 2 g |
| NaCl | 5 g |
| K₂CO₃ | 2 g |
| Humanes Serum Albumin | 2 g |
| Harnstoff | 1 g |
| Lactoferrin | 1 mg |
| mit destilliertem Wasser auf 1000 ml auffüllen | |
| pH-Wert auf 7 einstellen | |

Für die photometrische Bestimmung wird als Negativkontrolle die Speichelimitationslösung in der in der vorstehenden Tabelle angeführten Zusammensetzung und als Positivkontrolle Kalibratorlösung 1 verwendet. Das gewonnene Speichelvolumen beträgt 7 ml.

### Ausführungsbeispiel 6

Bevor der Proband 2 ml Speichelsammellösung mit der in der in der Tabelle aufgezeigten Zusammensetzung gurgelt, spült er den Mund mit destilliertem Wasser um eventuell in der Mundhöhle vorliegende Speisereste oder andere störende Komponenten zu entfernen.

### Speichelsammellösung:

| | |
|---|---|
| Natriumdihydrogencitrat | 1 g |
| Natriumcitrat Tribasic Monocitrat | 0,2 g |
| Testosteron | 10 ng |
| auf ein Volumen von 1000 ml mit destilliertem Wasser auffüllen | |
| einen pH-Wert von 4,2 einstellen | |

Nach einer Minute gurgeln mit der Speichelsammellösung wird das Speichel-Speichelsammellösungsgemisch im Behälter 1 gesammelt, wobei der Abstand des schlauchförmigen Fortsatz 9 vom Boden des Bodenteils 2 des Behälters 1 2 mm beträgt. Zudem ist im Behälter 1 im Bereich des schlauchförmigen Fortsatz 9 eine Filtrationseinrichtung 13 aus Nitrocellulose angeordnet. Die Porengröße der Filtrationseinrichtung 13 beträgt 40 µm.

Das Speichel-Speichelsammellösungsgemisch wird in ein evakuiertes Sammelgefäß, welches mit der in der Tabelle angeführten Lösung beschichtet ist, transferiert.

### Lösung mit Reagenzien:

| | |
|---|---|
| Kaliumsorbat | 0,5 g/l |
| Kalziumchlorid | 1,5 g/l |

Das Sammelgefäß mit dem darin enthaltenen Speichel-Speichelsammellösungsgemisch wird gekippt und bei 1.600 g für 8 Minuten bei Raumtemperatur zentrifugiert. Das Pellet wird verworfen und im Überstand Testosteron immunologisch mittels Elisa bestimmt. Zudem wird die Konzentration des Testosteron vor dem Spülen der Mundhöhle mit Speichelsammellösung aus dem Speichel bestimmt, um physiologisch vorliegendes Testosteron zu berücksichtigen. Im Speichel dieses lässt sich vor Beginn des erfindungsgemäßen Verfahrens kein Testosteron nachweisen.

Um eine Quantifizierung zu ermöglichen wird parallel mittels Elisa die Testosteronkonzentraion von Kalibratorlösungen 1 bis 5 mit der in der nachstehenden Tabelle angeführten Zusammensetzung ebenfalls gemessen.

### Kalibratorlösungen:

| | Kalibratorlösung 1 | Kalibratorlösung 2 | Kalibratorlösung 3 | Kalibratorlösung 4 | Kalibratorlösung 5 |
|---|---|---|---|---|---|
| Speichelsammellösung | 99,95 % | 74,95 % | 49,975 % | 25 % | 0 % |
| Speichelimitationslösung | 0% | 25 % | 49,975 % | 74,95 % | 99,95 % |
| Natriumazid | 0,05 % | 0,05 % | 0,05 % | 0,05 % | 0,05 % |

Die für die Herstellung der Kalibratorlösung verwendete Speichelsammellösung weist die in Ausführungsbeispiel 6 angeführte Zusammensetzung und die Speichelimitationslösung weist die in der nachfolgenden Tabelle angeführte Zusammensetzung auf

### Speichelimitationslösung:

| | |
|---|---|
| NaHCO₃ | 4,2 g |
| NaCl | 0,5 g |
| K₂CO₃ | 0,2 g |
| Humanes Serum Albumin | 250 mg |
| Harnstoff | 150 mg |
| Lactoferrin | 0,1 mg |
| Gelatine | 500 mg |
| mit destilliertem Wasser auf 1000 ml auffüllen | |
| pH-Wert auf 6,5 einstellen | |

Für den immunologischen Nachweis werden zwei Kontrollen verwendet. Kontrolle 1 weist eine Konzentration von 20 ng/ml und Kontrolle 2 von 60 ng/ml auf. Das entnommene Speichelvolumen beträgt 3 ml.

### Ausführungsbeispiel 7

Bevor der Proband 3 ml Speichelsammellösung mit der in der in der Tabelle aufgezeigten Zusammensetzung gurgelt, spült er den Mund mit destilliertem Wasser um eventuell in der Mundhöhle vorliegende Speisereste oder andere störende Komponenten zu entfernen.

### Speichelsammellösung:

| | |
|---|---|
| Natriumdihydrogencitrat | 7 g |
| Natriumcitrat Tribasic Monocitrat | 2 g |
| Vitamin B9 | 5 µg |
| auf ein Volumen von 1000 ml mit destilliertem Wasser auffüllen | |
| einen pH-Wert von 4,2 einstellen | |

Nach einer Minute gurgeln mit der Speichelsammellösung wird das Speichel-Speichelsammellösungsgemisch im Behälter 1 gesammelt, wobei der Abstand des schlauchförmigen Fortsatz 9 vom Boden des Bodenteils 2 des Behälters 2 einen Millimeter beträgt. Zudem ist im Behälter 1 im Bereich des schlauchförmigen Fortsatz 9 eine Filtrationseinrichtung 13 aus Zellulosetriacetat angeordnet. Die Porengröße der Filtrationseinrichtung 13 beträgt 80 µm.

Das Speichel-Speichelsammellösungsgemisch wird in ein evakuiertes Sammelgefäß, welches mit der in der Tabelle angeführten Lösung beschichtet ist, transferiert.

### Lösung mit Reagenzien:

| | |
|---|---|
| Kaliumbenzoat | 0,5 g/l |
| Ammoniumsulfat | 1,5 g/l |

Das Sammelgefäß mit dem darin enthaltenen Speichel-Speichelsammellösungsgemisch wird gekippt und bei 1.500 g für 15 Minuten bei Raumtemperatur zentrifugiert. Das Pellet wird verworfen und der Überstand wird einer HPLC zugeführt und die Konzentration mittels der in der Apparatur installierten Software bestimmt.

Um eine Quantifizierung zu ermöglichen werden die Kalibratorlösungen 1 bis 5 mit der in der nachstehenden Tabelle angeführten Zusammensetzung ebenfalls in der HPLC gemessen.

### Kalibratorlösungen:

| | Kalibratorlösung 1 | Kalibratorlösung 2 | Kalibratorlösung 3 | Kalibratorlösung 4 | Kalibratorlösung 5 |
|---|---|---|---|---|---|
| Speichelsammellösung | 99,95 % | 74,95 % | 49,975 % | 25 % | 0 % |
| Speichelimitationslösung | 0% | 25 % | 49,975 % | 74,95 % | 99,95 % |
| Natriumazid | 0,05 % | 0,05 % | 0,05 % | 0,05 % | 0,05 % |

Die für die Herstellung der Kalibratorlösung verwendete Speichelsammellösung weist die in Ausführungsbeispiel 7 angeführte Zusammensetzung und die Speichelimitationslösung weist die in der nachfolgenden Tabelle angeführte Zusammensetzung auf.

### Speichelimitationslösung:

| | |
|---|---|
| NaHCO₃ | 2,2 g |
| CaCl₂ | 1g |
| K₂CO₃ | 0,02 g |
| Humanes Serum Albumin | 250 mg |
| Harnstoff | 150mg |
| Gelatine | 500 mg |
| mit destilliertem Wasser auf 1000 ml auffüllen | |
| pH-Wert auf 5,5 einstellen | |

Für die HPLC Bestimmung werden ebenfalls gemäß den Vorschriften der Apparatur Positivkontrollen bestimmt.

Die Ausführungsbeispiele zeigen mögliche Ausführungsvarianten des Verfahrens zum Sammeln von Speichel, der Speichelsammellösung, des Behälters, des verschließbaren Sammelgefäßes, der Speichelimitationslösung, der Kalibratorlösungen und des Testkits, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mitumfasst.

Der Ordnung halber sei abschließend darauf hingewiesen, dass zum besseren Verständnis des Aufbaus des Behälters diese bzw. deren Bestandteile teilweise unmaßstäblich und/oder vergrößert und/oder verkleinert dargestellt wurden.

Die den eigenständigen erfinderischen Lösungen zugrunde liegende Aufgabe kann der Beschreibung entnommen werden.

### Bezugszeichenaufstellung

- 1: Behälter
- 2: Bodenteil
- 3: Deckelteil
- 4: Innenseite
- 5: Außenseite

- 6: Ausnehmung
- 7: Transfereinrichtung
- 8: Penetrationsvorrichtung
- 9: Fortsatz
- 10: Ende

- 11: Ende
- 12: Abstand
- 13: Filtrationseinrichtung

## Patentansprüche

1. Speichelsammellösung umfassend ein Mittel mit gustatorisch stimulierenden Eigenschaften, ein Agens und Wasser, und einen Farbstoff **dadurch gekennzeichnet, dass** der Farbstoff Tartrazin und/oder Chinolingelb ist, wobei dessen/deren Konzentration aus einem Bereich mit einer unteren Grenze von 0,0001 %, insbesondere 0,005 %, vorzugsweise 0,1 %, und einer oberen Grenze von 5 %, insbesondere 1 %, vorzugsweise 0,5 %, ausgewählt ist/sind.

2. Speichelsammellösung nach Anspruch 1, **dadurch gekennzeichnet, dass** das Mittel mit gustatorisch stimulierenden Eigenschaften aus einer Gruppe von an- und/oder organischen Genusssäuren und/oder deren Salzen oder Mischungen daraus ausgewählt ist.

3. Speichelsammellösung nach Anspruch 2, **dadurch gekennzeichnet, dass** die Genusssäuren aus einer Gruppe umfassend Phosphorsäure, Milchsäure, Zitronensäure, Ascorbinsäure, ausgewählt sind.

4. Speichelsammellösung nach Anspruch 1, **dadurch gekennzeichnet, dass** das Mittel mit gustatorischen Eigenschaften ein Wirkstoff, wie z.B. Pilocarpin, ist.

5. Speichelsammellösung nach einem der Ansprüche 1 bis 4, **dadurch gekennzeichnet, dass** die Konzentration des Mittels mit gustatorisch stimulierenden Eigenschaften aus einem Bereich mit einer unteren Grenze von 0,0005 %, insbesondere 0,01 %, vorzugsweise 1 %, und einer oberen Grenze von 10 %, insbesondere 5 %, vorzugsweise 2 %, ausgewählt ist.

6. Speichelsammellösung nach einem der Ansprüche 1 bis 5, **dadurch gekennzeichnet, dass** der Farbstoff eine Gelbfärbung verursacht und wasserlöslich ist ist.

7. Speichelsammellösung nach einem der Ansprüche 1 bis 6, **dadurch gekennzeichnet, dass** ein Agens im Sinne eines Medizinprodukts, in-vitro Diagnostikums und/oder Pharmazeutikums zugesetzt ist.

8. Speichelsammellösung nach einem der Ansprüche 1 bis 7, **dadurch gekennzeichnet, dass** ein Agens, welches mit routinemäßig verwendeten Labormessgeräten bestimmt werden kann, beigemengt ist.

9. Speichelsammellösung nach Anspruch 8, **dadurch gekennzeichnet, dass** als Agens zumindest ein Vertreter ausgewählt aus einer Gruppe umfassend Bilirubin, Acetaminophen, saure Phosphatase, Albumin, Kreatinin, Alkohol, alkalische Phosphatase, Alaninaminotransferase, Ammoniak, Amylase, Aspartataminotransferase, Bilirubin, unkonjugiert und konjugiert, Harnstoff, Calcium, Bicarbonat, Cholinesterase, LDL- bzw. HDL Cholesterin, Lysozym, Amylase, Creatinkinase, Creatinkinase - MB, Chlorid, Carbamazepin, Creatinin, C-reaktives Protein, direktes Bilirubin, Digoxin, Kohlendioxid, Eisen, Gamma-Glutamyltransferase, Glukose, magnetisches HDL-Cholesterinreagens, Kalium, Lactat, Lactatdehydrogenase, Lithium, Lipase, anorganische Verbindungen, wie z.B. Spurenelemente, Mineralstoffe, wie Magnesium, Natrium, Neugeborenen-Bilirubin, Phenobarbital, Phosphor, Phenytoin, Primidon, Liquorprotein, Salicylat, Gesamt-Bilirubin, Theophyllin, Reagens für totale Eisenbindungskapazität, Gesamtprotein, Triglycerid, Urinprotein, Harnsäure, β-Microglobulin, Corticosteroid- bzw. Sexualhormon bindendes Globulin, Thiocyanat, Transferrin, diverse Lipide, Lipoproteine, Proteine, Kohlenhydrate, insbesondere wasserlösliche Kohlenhydrate, Vitamine, insbesondere wasserlösliche Vitamine, wie z.B. Vitamine der B-, C- und F-Gruppe, anorganische Verbindungen, wie z.B. Spurenelemente, Mineralstoffe, Hormone, wie z.B. Aldosteron, Androstenedion, Cortisol, Dihydroepiandrosteron, bzw. -sulfate, Östradiol, Östriol, Progesteron, Testosteron, bzw. Mischungen daraus zugesetzt ist.

10. Speichelsammellösung nach einem der Ansprüche 1 bis 9, **dadurch gekennzeichnet, dass** die Konzentration des Agens aus einem Bereich mit einer unteren Grenze von 0,0000000001 %, insbesondere 0,00005 %, vorzugsweise 0,001 %, und einer oberen Grenze von 10 %, insbesondere 5 %, vorzugsweise 2 %, ausgewählt ist.

11. Speichelsammellösung nach einem der Ansprüche 1 bis 10, **dadurch gekennzeichnet, dass** ein Aromastoff und/oder Geschmacksverstärker zugesetzt ist.

12. Speichelsammellösung nach Anspruch 11, **dadurch gekennzeichnet, dass** der Aromastoff aus einer Gruppe von Zuckerarten bzw. Zuckeraustauschstoffe, wie z.B. Saccharose, Maltose, Fructose, und/oder Süßungsmittel, wie z.B. Saccharin, Aspartam, und/oder Mischungen daraus ausgewählt ist.

13. Speichelsammellösung nach Anspruch 11 oder 12, **dadurch gekennzeichnet, dass** der Aromastoff und/oder Geschmacksverstärker natürlichen und/oder synthetischen Ursprungs aus einer Gruppe umfassend Früchte, wie Apfel, Himbeere, Kirsche, Erdbeere, Zitrone, Limone, Orange, Lakritze, Kräuter, Chinin, Koffein, Teein, bzw. Mischungen daraus ausgewählt ist.

14. Speichelsammellösung nach einem der Ansprüche 11 bis 13, **dadurch gekennzeichnet, dass** die Konzentration des Aromastoffes und/oder Geschmacksverstärkers aus einem Bereich mit einer unteren Grenze von 0,001 %, insbesondere 0,01 %, vorzugsweise 0,1 %, und einer oberen Grenze von 10 %, insbesondere 5 %, vorzugsweise 1 %, ausgewählt ist.

15. Speichelsammellösung nach einem der Ansprüche 1 bis 14, **dadurch gekennzeichnet, dass** der pH-Wert aus einem Bereich mit einer unteren Grenze von 3, vorzugsweise 3,5, insbesondere 4 und einer oberen Grenze von 6, vorzugsweise 5, insbesondere 4,5 ausgewählt ist.

16. Verwendung der Speichelsammellösung nach einem der Ansprüche 1 bis 15 zur Anregung des Speichelflusses und Quantifizierung des Speichelvolumens.

17. Verfahren zum Sammeln von Speichel aus der Mundhöhle zur Detektion eines Analyten umfassend die Schritte (a) Reinigung der Mundhöhle, (b) Anregung der Speichelsekretion mit einer Speichelsammellösung nach einem der Ansprüche 1 bis 15, (c) Entfernen des Speichel-Speichelsammellösungsgemisches aus der Mundhöhle und Sammeln in einem Behälter (1), (d) Überführen des Speichel-Speichelsammellösungsgemisch in ein verschließbares Sammelgefäß.

18. Verfahren nach Anspruch 17, **dadurch gekennzeichnet, dass** durch die Speichelsammellösung mit gustatorisch stimulierenden Eigenschaften die Sekretion von Speichel erhöht wird.

19. Verfahren nach Anspruch 17 oder 18, **dadurch gekennzeichnet, dass** das Speichel-Speichelsammellösungsgemisch über eine im oder am Behälter (1) integrierte Transfereinrichtung (7) in das verschließbare Sammelgefäß überführt wird.

20. Verfahren nach einem der Ansprüche 17 bis 19, **dadurch gekennzeichnet, dass** aus dem Speichel-Speichelsammellösungsgemisch über eine Filtrationseinrichtung (13) im Behälter
(1) partikuläre Bestandteile entfernt werden.

21. Verfahren nach einem der Ansprüche 17 bis 20, **dadurch gekennzeichnet, dass** ein verschließbares Sammelgefäß mit einer Lösung mit konservierenden und reduzierenden Eigenschaften verwendet wird.

22. Verfahren nach einem der Ansprüche 17 bis 21, **dadurch gekennzeichnet, dass** die Menge des Speichels quantitativ bestimmt wird.

23. Verfahren nach Anspruch 22, **dadurch gekennzeichnet, dass** die Quantifizierung des Speichels unter Verwendung zumindest einer Kalibratorlösung durchgeführt wird.

24. Verfahren nach einem der Ansprüche 17 bis 23, **dadurch gekennzeichnet, dass** zur quantitativen Bestimmung die Konzentration und/oder Extinktion des Farbstoffes und gegebenenfalls Agens des Speichel-Speichelsammellösungsgemisches mit der Konzentration und/oder Extinktion des Farbstoffes und gegebenenfalls Agens, welche im Speichel vor dessen Entnahme vorliegt, verglichen wird.

25. Verfahren nach einem der Ansprüche 17 bis 24, **dadurch gekennzeichnet, dass** das in einem evakuierten Sammelgefäß vorliegende Speichel-Speichelsammellösungsgemisch zentrifugiert wird, und gegebenenfalls vorher gemischt wird.

26. Verfahren nach einem der Ansprüche 17 bis 25, **dadurch gekennzeichnet, dass** das Speichel-Speichelsammellösungsgemisch für Analysen verwendet wird.

27. Verfahren nach Anspruch 26, **dadurch gekennzeichnet, dass** die Analysen in einem standardisierten Labormessgerät durchgeführt werden.

## Claims

1. Saliva-collecting solution comprising a substance with taste-stimulating properties and an agent, water and a dye, **characterised in that** the dye is tartrazine and/or chinoline yellow, and its concentration is selected from a range with a lower limit of 0.0001 %, in particular 0.005 %, preferably 0.1 %, and an upper limit of 5 %, in particular 1 %, preferably 0.5 %.

2. Saliva-collecting solution as claimed in claim 1, **characterised in that** the substance with taste-stimulating properties is selected from a group of inorganic and/or organic edible acids and/or salts or mixtures thereof.

3. Saliva-collecting solution as claimed in claim 2, **characterised in that** the edible acids are selected from a group comprising phosphoric acid, lactic acid, citric acid, ascorbic acid.

4. Saliva-collecting solution as claimed in claim 1, **characterised in that** the substance with taste-stimulating properties is an active substance such as pilocarpine, for example.

5. Saliva-collecting solution as claimed in one of claims 1 to 4, **characterised in that** the concentration of the substance with taste-stimulating properties is selected so as to be within a range with a lower limit of 0.0005 %, in particular 0.01 %, preferably 1 %, and an upper limit of 10 %, in particular 5 %, preferably 2 %.

6. Saliva-collecting solution as claimed in one of claims 1 to 5, **characterised in that** the dye causes a yellow colouration and is water-soluble.

7. Saliva-collecting solution as claimed in one of claims 1 to 6, **characterised in that** an agent in the sense of a medicinal product, in-vitro diagnostic reagent and/or pharmaceutical product is added.

8. Saliva-collecting solution as claimed in one of claims 1 to 7, **characterised in that** an agent which can be detected by laboratory measuring equipment used as a matter of routine is added.

9. Saliva-collecting solution as claimed in claim 8, **characterised in that** the added agent is a representative selected from a group comprising bilirubin, acetaminophen, acidic phosphatase, albumin, creatinine, alcohol, alkaline phosphatase, alaninamino-transferase, ammonia, amylase, aspartatamino-transferase, bilirubin, unconjugated and conjugated, urea, calcium, bicarbonate, cholinesterase, LDL- or. HDL cholesterine, lysozyme, amylase, creatine kinase, creatine kinase - MB, chloride, carbamazepine, creatinine, C-reactive protein, direct bilirubin, digoxin, carbon dioxide, iron, gamma-glutamyl transferase, glucose, magnetic HDL-cholesterine reagent, potassium, lactate, lactate dehydrogenase, lithium, lipase, inorganic compounds, such as for example trace elements, minerals, such as magnesium, sodium, new-born bilirubin, phenobarbital, phosphorous, phenytoin, primidone, liquor protein, salicylate, whole bilirubin, theophylline, reagent for whole iron binding capacity, whole protein, triglyceride, urine protein, uric acid, β-microglobulin, corticosteroid- or sex hormone-binding globulin, thiocyanate, transferrin, various lipids, lipoproteins, proteins, carbohydrates, in particular water-soluble carbohydrates, vitamins, in particular water-soluble vitamins, such as for example vitamins of the B, C and F group, inorganic compounds, such as for example trace elements, minerals, hormones, such as for example aldosterone, androstenedione, cortisone, dihydroepiandrosterone, or-sulphates, oestradiol, oestriol, progesterone, testosterone, or mixtures thereof.

10. Saliva-collecting solution as claimed in one of claims 1 to 9, **characterised in that** the concentration of the agent is selected from a range with a lower limit of 0.0000000001%, in particular 0.00005 %, preferably 0.001 %, and an upper limit of 10 %, in particular 5 %, preferably 2 %.

11. Saliva-collecting solution as claimed in one of claims 1 to 10, **characterised in that** a flavouring and/or flavour enhancer is added.

12. Saliva-collecting solution as claimed in claim 11, **characterised in that** the flavouring is selected from a group of sugar types or sugar substitutes, such as for example saccharose, maltose, fructose, and/or sweeteners, such as for example saccharin, aspartame, and/or mixtures thereof.

13. Saliva-collecting solution as claimed in claim 11 or 12, **characterised in that** the flavouring or flavour enhancer may be of natural and/or synthetic origin and is selected from a group comprising fruits, such as apples, raspberries, cherries, strawberries, lemon, lime, orange, liquorice, herbs, quinine, caffeine, tannin, or mixtures thereof.

14. Saliva-collecting solution as claimed in one of claims 11 to 13, **characterised in that** the concentration of flavouring and/or flavour enhancer is selected from a range with a lower limit of 0.001 %, in particular 0.01 %, preferably 0.1 %, and an upper limit of 10 %, in particular 5 %, preferably 1 %.

15. Saliva-collecting solution as claimed in one of claims 1 to 14, **characterised in that** the pH value is selected from a range with a lower limit of 3, preferably 3.5, in particular 4, and an upper limit of 6, preferably 5, in particular 4.5.

16. Use of the saliva -collecting solution as claimed in one of claims 1 to 15 for stimulating the flow of saliva and quantifying the volume of saliva.

17. Method of collecting saliva from the oral cavity in order to detect a test substance, comprising the steps of (a) cleaning the oral cavity, (b) stimulating saliva secretion with a saliva-collecting solution as claimed in one of claims 1 to 15, (c) removing the saliva-collecting solution mixture from the oral cavity and collecting it in a container (1), (d) transferring the saliva-collecting solution mixture to a sealable collection vessel.

18. Method as clamed in claim 17, **characterised in that** the secretion of saliva is increased by the saliva-collecting solution with taste-stimulating properties.

19. Method as claimed in claim 17 or 18, **characterised in that** the saliva-collecting solution mixture is transferred via a transfer mechanism (7) integrated in or on the container (1) into the sealable collection vessel.

20. Method as claimed in one of claims 17 to 19, **characterised in that** particulate elements are removed from the saliva-collecting solution mixture by means of a filtration unit (13) in the container (1).

21. Method as claimed in one of claims 17 to 20 **characterised in that** a sealable collection vessel is used with a solution having preserving and reducing properties.

22. Method as claimed in one of claims 17 to 21, **characterised in that** the quantity of saliva is quantitatively determined.

23. Method as claimed in claim 22, **characterised in that** the saliva is quantified using at least one calibrator solution.

24. Method as claimed in one of claims 17 to 23, **characterised in that** in order to make a quantitative determination, the concentration and/or extinction of the dye and optionally the agent of the saliva-collecting solution mixture is compared with the concentration and/or extinction of the dye and optionally the agent present in the saliva prior to taking the sample.

25. Method as claimed in one of claims 17 to 24, **characterised in that** the saliva-collecting solution mixture is centrifuged and optionally mixed beforehand in an evacuated collection vessel.

26. Method as claimed in one of claims 17 to 25, **characterised in that** the saliva-collecting solution mixture is used for conducting tests.

27. Method as claimed in claim 26, **characterised in that** the tests are conducted in standardised laboratory measuring equipment.

## Revendications

1. Solution collectrice de salive, comprenant un produit avec des propriétés stimulatrices du goût, un agent et de l'eau et un colorant, **caractérisée en ce que** le colorant est de la tartrazine et/ou du jaune de quinoléine, sa/leurs concentration(s) étant choisie(s) dans un intervalle avec une limite inférieure de 0,0001 %, en particulier de 0,005 %, de préférence de 0,1 %, et une limite supérieure de 5 %, en particulier de 1 %, de préférence de 0,5 %.

2. Solution collectrice de salive selon la revendication 1, **caractérisée en ce que** le produit avec des propriétés stimulatrices du goût est choisi parmi un groupe d'acides stimulants inorganiques et/ou organiques et/ou parmi leurs sels ou des mélanges de ceux-ci.

3. Solution collectrice de salive selon la revendication 2, **caractérisée en ce que** les acides stimulants sont choisis parmi un groupe comprenant l'acide phosphorique, l'acide lactique, l'acide citrique, l'acide ascorbique.

4. Solution collectrice de salive selon la revendication 1, **caractérisée en ce que** le produit avec des propriétés gustatives est un principe actif, comme par exemple la pilocarpine.

5. Solution collectrice de salive selon l'une quelconque des revendications 1 à 4, **caractérisée en ce que** la concentration du produit avec des propriétés stimulatrices du goût est choisie dans un intervalle avec une limite inférieure de 0,0005 %, en particulier de 0,01 %, de préférence de 1 %, et une limite supérieure de 10 %, en particulier 5 %, de préférence 2%.

6. Solution collectrice de salive selon l'une quelconque des revendications 1 à 5, **caractérisée en ce que** le colorant provoque une coloration en jaune et est soluble dans l'eau.

7. Solution collectrice de salive selon l'une quelconque des revendications 1 à 6, **caractérisée en ce qu'**un agent, au sens d'un produit médical, d'un produit de diagnostic in-vitro et/ou d'un produit pharmaceutique, est ajouté.

8. Solution collectrice de salive selon l'une quelconque des revendications 1 à 7, **caractérisée en ce qu'**un agent qui peut être déterminé avec les instruments de mesure de laboratoire utilisés habituellement, est ajouté.

9. Solution collectrice de salive selon la revendication 8, **caractérisée en ce que** comme agent, au moins un représentant choisi parmi un groupe comprenant la bilirubine, l'acétaminophène, la phosphatase acide, l'albumine, la créatinine, l'alcool, la phosphatase alcaline, l'alaninaminotransférase, l'ammoniac, l'amylase, l'aspartate aminotransférase, la bilirubine, non conjuguée et conjuguée, l'urée, le calcium, le bicarbonate, la cholinestérase, les cholestérols LDL, respectivement HDL, le lysozyme, l'amylase, la créatine kinase, la créatine kinase - MB, le chlorure, la carbamazépine, la créatinine, la protéine C-réactive, la bilirubine directe, la digoxine, le dioxyde de carbone, le fer, la gamma-glutamyl transférase, le glucose, le réactif magnétique au cholestérol HDL, le potassium, le lactate, la lactate déshydrogénase, le lithium, la lipase, des composés inorganiques comme par exemple des oligoéléments, des composés minéraux comme le magnésium, le sodium, la bilirubine de nouveau-né, le phénobarbital, le phosphore, la phénytoïne, la primidone, la protéine de liquides physiologique, le salicylate, la bilirubine totale, la théophylline, le réactif pour la capacité totale de liaison du fer, le total des protéines, le triglycéride, la protéine urinaire, l'acide urique, la β-microglobuline, la globuline de liaison des hormones corticostéroïdes, respectivement sexuelles, le thiocyanate, la transferrine, divers lipides, les lipoprotéines, les protéines, les hydrates de carbone, en particulier les hydrates de carbone solubles dans l'eau, les vitamines, en particulier les vitamines solubles dans l'eau, comme par exemple les vitamines des groupes B, C et F, des composés inorganiques, comme par exemple des oligoéléments, des composés minéraux, des hormones, comme par exemple l'aldostérone, l'androstènedione, le cortisol, la dihydroépiandrostérone, respectivement ses sulfates, l'estradiol, l'estriol, la progestérone, la testostérone, respectivement des mélanges de ceux-ci, est ajouté.

10. Solution collectrice de salive selon l'une quelconque des revendications 1 à 9, **caractérisée en ce que** la concentration de l'agent est choisie dans un intervalle avec une limite inférieure de 0,0000000001 %, en particulier de 0,00005 %, de préférence de 0,001 %, et une limite supérieure de 10 %, en particulier 5 %, de préférence 2 %.

11. Solution collectrice de salive selon l'une quelconque des revendications 1 à 10, **caractérisée en ce qu'**un aromate et/ou un exhausteur de goût est ajouté.

12. Solution collectrice de salive selon la revendication 11, **caractérisée en ce que** l'aromate est choisi parmi un groupe de sucres, respectivement d'édulcorants, comme par exemple le saccharose, le maltose, le fructose, et/ou des édulcorants, comme par exemple la saccharine, l'aspartame, et/ou des mélanges de ceux-ci.

13. Solution collectrice de salive selon la revendication 11 ou 12, **caractérisée en ce que** l'aromate et/ou l'exhausteur de goût, d'origine naturelle et/ou synthétique, sont choisis dans un groupe comprenant des fruits, comme des pommes, de framboises, des cerises, des fraises, des citrons, des limes, des oranges, le réglisse, les herbes, la quinine, la caféine, la théine, respectivement des mélanges de ces derniers.

14. Solution collectrice de salive selon l'une quelconque des revendications 11 à 13, **caractérisée en ce que** la concentration de l'aromate et/ou de l'exhausteur de goût est choisie dans un intervalle avec une limite inférieure de 0,001 %, en particulier de 0,01 %, de préférence de 0,1 %, et une limite supérieure de 10 %, en particulier de 5 %, de préférence de 1%.

15. Solution collectrice de salive selon l'une quelconque des revendications 1 à 14, **caractérisée en ce que** le pH est choisi dans un intervalle avec une limite inférieure de 3, de préférence de 3,5, en particulier de 4 et une limite supérieure de 6, de préférence de 5, en particulier de 4,5.

16. Utilisation de la solution collectrice de salive selon l'une quelconque des revendications 1 à 15 pour la stimulation de la salivation et pour la quantification du volume de salive.

17. Procédé de collecte de la salive depuis la cavité buccale pour la détection d'un analyte, comprenant les étapes (a) nettoyage de la cavité buccale, (b) stimulation de la sécrétion de salive avec une solution collectrice de salive selon l'une quelconque des revendications 1 à 15, (c) extraction du mélange de salive et de solution collectrice de salive de la cavité buccale et collecte dans un récipient (1), (d) transfert du mélange de salive et de solution collectrice de salive dans un récipient collecteur pouvant être fermé.

18. Procédé selon la revendication 17, **caractérisée en ce que** par la solution collectrice de salive avec des propriétés gustatives stimulatrices, la sécrétion de la salive est accrue.

19. Procédé selon la revendication 17 ou 18, **caractérisée en ce que** le mélange de salive et de solution collectrice de salive est transféré dans le récipient collecteur pouvant être fermé par un dispositif de transfert (7) intégré dans ou sur le récipient (1).

20. Procédé selon l'une quelconque des revendications 17 à 19, **caractérisé en ce que** les composants en particules sont éliminés du mélange de salive et de solution collectrice de salive par un dispositif de filtrage (13) dans le récipient (1).

21. Procédé selon l'une quelconque des revendications 17 à 20, **caractérisé en ce qu'**un récipient collecteur pouvant être fermé est utilisé avec une solution avec des propriétés de conservation et de réduction.

22. Procédé selon l'une quelconque des revendications 17 à 21, **caractérisé en ce que** la quantité de la salive est déterminée quantitativement.

23. Procédé selon la revendication 22, **caractérisé en ce que** la quantification de la salive est effectuée moyennant l'utilisation d'au moins une solution de calibrage.

24. Procédé selon l'une quelconque des revendications 17 à 23, **caractérisé en ce que** pour la détermination quantitative, la concentration et/ou l'extinction du colorant et, le cas échéant, celles de l'agent du mélange de salive et de solution collectrice de salive sont comparées à la concentration et/ou à l'extinction du colorant et le cas échéant de l'agent, qui règnent dans la salive avant son prélèvement.

25. Procédé selon l'une quelconque des revendications 17 à 24, **caractérisé en ce que** le mélange de salive et de solution collectrice de salive présent dans un récipient collecteur évacué est centrifugé et, le cas échéant, mélangé auparavant.

26. Procédé selon l'une quelconque des revendications 17 à 25, **caractérisé en ce que** le mélange de salive et de solution collectrice de salive est utilisé pour des analyses.

27. Procédé selon la revendication 26, **caractérisé en ce que** les analyses sont effectuées dans un instrument de mesure de laboratoire standardisé.
